(19) Europäisches Patentamt / European Patent Office / Office européen des brevets

(11) **EP 3 091 463 A1**

(12) **EUROPEAN PATENT APPLICATION**

(43) Date of publication:
**09.11.2016 Bulletin 2016/45**

(51) Int Cl.:
***G06F 19/22*** *(2011.01)*

(21) Application number: **16168108.5**

(22) Date of filing: **03.05.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.05.2015 JP 2015096031**

(71) Applicant: **SYSMEX CORPORATION Kobe-shi Hyogo 651-0073 (JP)**

(72) Inventor: **Tsuruoka, Rena Hyogo, 651-0073 (JP)**

(74) Representative: **Hoffmann Eitle Patent- und Rechtsanwälte PartmbB Arabellastraße 30 81925 München (DE)**

(54) **APPARATUS AND METHOD FOR ESTIMATING HEAT TREATMENT CONDITION, AND COMPUTER PROGRAM**

(57)     Disclosed is an apparatus configured to estimate a heat treatment condition, the apparatus including: an input unit configured to receive an input of information of one to three heat treatment conditions selected from the group consisting of: heating time; heating temperature; kind of a buffer agent; and salt concentration of a sample which contains the nucleic acid and the buffer agent, and an input of information of a desired average nucleotide length; and a controller programmed to perform operations comprising: on the basis of the information the inputs of which have been received by the input unit, estimating a heat treatment condition selected from the group consisting of: the heating time; the heating temperature; the kind of the buffer agent; and the salt concentration of the sample.

FIG. 7

EP 3 091 463 A1

**Description**

CROSS REFERENCE TO RELATED APPLICATIONS

This application claims priority from prior Japanese Patent Application No. 2015-096031, filed on May 8, 2015, entitled "Apparatus and method for estimating heat treatment condition, system and method for fragmenting nucleic acid, and computer program", the entire content of which is incorporated herein by reference.

FIELD OF THE INVENTION

The present invention relates to an apparatus and a method for estimating a heat treatment condition, a system and a method for fragmenting nucleic acid, and a computer program.

BACKGROUND

As a method for fragmenting nucleic acid, a method described in Non-Patent Literature "Fragmentation of Genomic DNA using Microwave Irradiation" by Yu Yang et al., Journal of Biomolecular Techniques, 2013, vol. 24, pp.98-103 has been proposed, for example. In the method described in Non-Patent Literature, a sample that contains DNA is irradiated with a microwave to heat-treat the sample. As a result of this, DNA is fragmented.

SUMMARY OF THE INVENTION

However, the length of the fragmented DNA depends on the heat treatment condition. Thus, it is not easy to predict a heat treatment condition for obtaining DNA having a desired length.
The present invention provides an apparatus and method for estimating a heat treatment condition, a system and a method for fragmenting nucleic acid, and a computer program.

(1) The present invention provides an apparatus for estimating a heat treatment condition, the apparatus comprising:

input means for receiving an input of information of one to three heat treatment conditions selected from the group consisting of: heating time; heating temperature; kind of a buffer agent; and salt concentration of a sample which contains the nucleic acid in the sample and the buffer agent, and an input of information of a desired average nucleotide length; and
control means, wherein
on the basis of the information the inputs of which have been received by the input means, the control means estimates a heat treatment condition selected from the group consisting of: the heating time; the heating temperature; the kind of the buffer agent; and the salt concentration of the sample.

(2) The apparatus of (1), wherein
the input means further receives an input of information regarding the kind of the nucleic acid.

(3) The apparatus of (1) or (2), wherein
the input means further receives an input of information regarding a nucleotide length of nucleic acid before being subjected to the heat treatment.

(4) The apparatus of any one of (1) to (3), wherein
the information of the heat treatment conditions includes a treatment condition library which defines correspondence relation among the kind of the buffer agent, the salt concentration of the sample, and the heating temperature or the heating time.

(5) The apparatus of (4), wherein
on the basis of the information the inputs of which have been received by the input means, the control means outputs a constant of speed of fragmentation of nucleic acid in the heat treatment, using the treatment condition library.

(6) The apparatus of (4) or (5), wherein
using the treatment condition library, the control means outputs information regarding a minimum nucleotide length of the nucleic acid fragments obtained in the heat treatment.

(7) The apparatus of any one of (1) to (6), further comprising:

> storage means having stored therein correspondence relation information which defines correspondence relation among the desired average nucleotide length, the heating time, the heating temperature, the kind of the buffer agent, and the salt concentration of the sample, wherein
> on the basis of the information the inputs of which have been received by the input means, and on the basis of the correspondence relation information stored in the storage means, the control means estimates a heat treatment condition selected from the group consisting of: the heating time; the heating temperature; the kind of the buffer agent; and the salt concentration of the sample.

(8) The apparatus of (7), wherein
the storage means has further stored therein a relational expression indicative of correlation among the desired average nucleotide length, the minimum nucleotide length, the nucleotide length of nucleic acid before being subjected to the heat treatment, energy for fragmentation of nucleic acid in the heat treatment, the heating temperature, and a start temperature of the heat treatment.

(9) The apparatus of (8), wherein
the relational expression is represented by Expression (I):

$$M = A + B \times \exp\{-H(T-D)\} \qquad\qquad (I)$$

(wherein, M represents the desired average nucleotide length, A represents the minimum nucleotide length, B represents the nucleotide length of nucleic acid before being subjected to the heat treatment, H represents a constant of energy for fragmentation of nucleic acid in the heat treatment, T represents the heating temperature, and D represents the start temperature of the heat treatment).

(10) The apparatus of (7), wherein
the storage means has further stored therein a relational expression indicative of correlation among the desired average nucleotide length, the minimum nucleotide length, the nucleotide length of nucleic acid before being subjected to the heat treatment, the speed of fragmentation of nucleic acid in the heat treatment, and the heating time.

(11) The apparatus of (10), wherein
the relational expression is represented by Expression (II):

$$M = A + B \times \exp\{-Kt\} \qquad\qquad (II)$$

(wherein, M represents the desired average nucleotide length, A represents the minimum nucleotide length, B represents the nucleotide length of nucleic acid before being subjected to the heat treatment, K represents a constant of the speed of fragmentation of nucleic acid in the heat treatment, and t represents the heating time).

(12) The apparatus of any one of (6) to (11), wherein
the storage means has further stored therein information regarding the minimum nucleotide length.

(13) The apparatus of (12), wherein
the minimum nucleotide length is 10 to 80 nucleotide length.

(14) The apparatus of any one of (1) to (13), wherein
the heating temperature is 90 to 200°C.

(15) The apparatus of any one of (1) to (14), further comprising
output means for outputting the heat treatment condition estimated by the control means.

(16) The apparatus of (15), wherein
on the basis of the information regarding the kind of the buffer agent, the information regarding the salt concentration of the sample, and the information regarding a condition selected from between the heating time and the heating temperature, the control means outputs, to the output means, information that indicates correspondence relation between the selected condition and an average nucleotide length of the nucleic acid fragments obtained through the heat treatment, and

on the basis of the information regarding the desired average nucleotide length inputted by a user on the basis of the information indicating the correspondence relation, the control means further estimates a heat treatment condition that allows obtainment of nucleic acid fragments having the desired average nucleotide length.

(17) The present invention provides a system for fragmenting nucleic acid, the system comprising:

the apparatus for estimating a heat treatment condition of any one of (1) to (16); and
a heating apparatus for heating a sample containing the nucleic acid, on the basis of information of the heat treatment condition estimated by the estimation apparatus.

(18) The present invention provides a method for estimating a heat treatment condition, the method comprising:

on the basis of
information of one to three heat treatment conditions selected from the group consisting of: heating time; heating temperature; kind of a buffer agent; and salt concentration of a sample which contains the nucleic acid in the sample and the buffer agent, and
information of the desired average nucleotide length,
estimating a heat treatment condition selected from the group consisting of: the heating time; the heating temperature; the kind of the buffer agent; and the salt concentration of the sample, wherein the heat treatment condition is for fragmenting, through heat treatment, into the nucleic acid fragments having the desired average nucleotide length.

(19) The method of (18), further comprising:

using, when estimating the heat treatment condition, correspondence relation information that defines correspondence relation among the heating time, the heating temperature, the kind of the buffer agent, and the salt concentration of the sample.

(20) The present invention provides a method for fragmenting nucleic acid, the method comprising:

on the basis of the heat treatment condition estimated according to the method of (18) or (19), heating the sample to fragment the nucleic acid into nucleic acid fragments having the desired average nucleotide length.

(21) The present invention provides a computer program for estimating a heat treatment condition, the computer program for causing a computer to function as:

input means for receiving an input of information of one to three heat treatment conditions selected from the group consisting of: heating time; heating temperature; kind of a buffer agent; and salt concentration of a sample which contains the nucleic acid in the sample and the buffer agent, and an input of information of a desired average nucleotide length; and
control means for estimating a heat treatment condition selected from the group consisting of: the heating time; the heating temperature; the kind of the buffer agent; and the salt concentration of the sample, on the basis of the information the inputs of which have been received by the input means.

According to the present invention, it is possible to provide an apparatus and a method for estimating a heat treatment condition, a system and a method for fragmenting nucleic acid using these, and a computer program, which can estimate in a simple manner a heat treatment condition when performing fragmentation of nucleic acid through heat treatment.

BRIEF DESCRIPTION OF THE DRAWINGS

FIG 1 is a perspective view explaining a nucleic acid fragmentation system;
FIG. 2 is a block diagram of an estimation apparatus;

FIG. 3 is a flow chart showing a process procedure performed by the nucleic acid fragmentation system;

FIG 4 is a flow chart showing a process procedure of a practical mode and a condition proposal mode;

FIG. 5 is a flow chart showing a process procedure of the temperature-constant mode in the practical mode;

FIG 6 is a schematic diagram showing one example of the data structure of a treatment condition library used in the practical mode;

FIG 7 is a flow chart showing a process procedure of the temperature-constant mode in the practical mode;

FIG 8 shows a display screen on display means;

FIG 9 shows a display screen on the display means;

FIG. 10 is a flow chart showing a process procedure of a time-constant mode in the practical mode;

FIG 11 is a flow chart showing a process procedure of the time-constant mode in the practical mode;

FIG. 12 is a flow chart showing a process procedure of the temperature-constant mode in the condition proposal mode;

FIG 13 shows a display screen on the display means;

FIG 14 shows a display screen on the display means;

FIG 15 is a schematic diagram showing one example of the data structure of a treatment condition library used in the condition proposal mode;

FIG. 16 shows a display screen on the display means;

FIG 17 is a flow chart showing a process procedure of the time-constant mode in the condition proposal mode;

FIG 18 is a photograph as a substitute for a drawing of an electrophoresis gel after electrophoresis in Example 1;

FIG. 19 is graphs each showing the relationship between a band and mobility in FIG 18;

FIG 20 is a graph of a calibration curve showing the relationship between the mobility and the logarithm value of nucleic acid fragment length;

FIG. 21 is a graph showing the relationship between the heating temperature and the logarithm value of the nucleic acid fragment length;

FIG 22 is a graph showing a result of curve fitting performed with respect to plotted data points of the nucleic acid fragment length and the heating temperature;

FIG. 23 is a photograph as a substitute for a drawing of an electrophoresis gel after electrophoresis in Example 3;

FIG 24 is a graph showing a result of curve fitting performed with respect to plotted data points of the nucleic acid fragment length and the heating time;

FIG. 25 is a photograph as a substitute for a drawing of an electrophoresis gel after electrophoresis in Example 5;

FIG. 26 is a graph showing a result of curve fitting performed with respect to plotted data points of the nucleic acid fragment length and the heating temperature;

FIG 27 is a photograph as a substitute for a drawing of an electrophoresis gel after electrophoresis in Example 6; and

FIG. 28 is a graph showing a result of curve fitting performed with respect to plotted data points of the nucleic acid fragment length and the heating time.

## DETAILED DESCRIPTION OF THE PREFERRED EMBODIMENTS

[Overall configuration of nucleic acid fragmentation system]

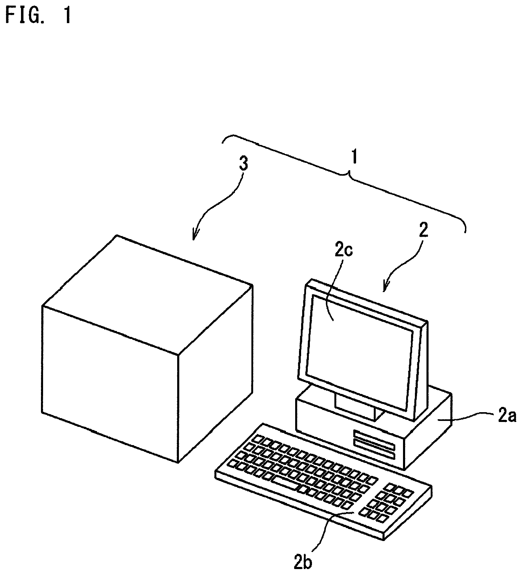

A nucleic acid fragmentation system will be described with reference to FIG. 1.

As shown in FIG 1, a nucleic acid fragmentation system 1 includes: an estimation apparatus 2; and a heating apparatus 3 communicably connected to the estimation apparatus 2. In the present embodiment, the estimation apparatus 2 is a computer system which includes a computer body 2a, an input device 2b, and a monitor 2c. The heating apparatus 3 is a microwave irradiation apparatus for heating a nucleic acid-containing sample, by irradiating the sample with a microwave. The input device 2b functions as input means for the estimation apparatus 2. The monitor 2c functions as display means for the estimation apparatus 2.

Examples of the input device 2b include a keyboard and the like. The monitor 2c may be a touch panel. In such a case, the monitor 2c is also used as the input device 2b. As the input device 2b, a stylus may further be used. Information is inputted via the input device 2b. Examples of the information to be inputted include: one to three heat treatment conditions selected from the group consisting of heating time, heating temperature, kind of buffer agent for the nucleic acid-containing sample, and salt concentration of the sample; and information of a desired average nucleotide length.

[Configuration of estimation apparatus]

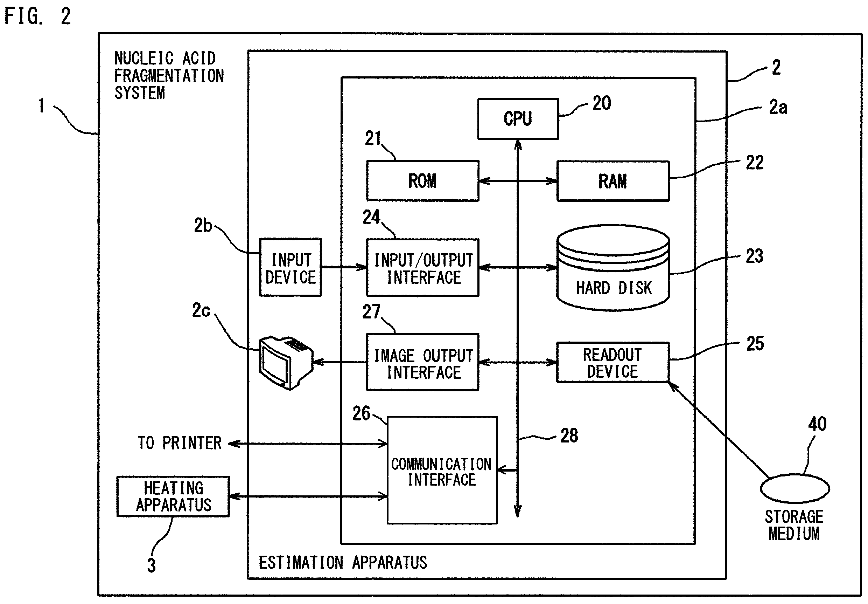

As shown in FIG 2, the computer body 2a of the estimation apparatus 2 includes a CPU (central processing unit) 20, a ROM (read only memory) 21, a RAM (random access memory) 22, a hard disk 23, an input/output interface 24, a readout device 25, a communication interface 26, and an image output interface 27. The CPU 20, the ROM 21, the RAM 22, the hard disk 23, the input/output interface 24, the readout device 25, the communication interface 26, and the image output interface 27 are data-communicably connected to each other by a bus 28. The CPU 20 functions as control means for the computer body 2a. The hard disk 23 functions as storage means for the computer body 2a.

The CPU 20 can execute computer programs stored in the ROM 21 and computer programs loaded into the RAM 22. By the CPU 20 executing application programs, the display means, the input means, the control means, and the storage means are realized in the computer system. Accordingly, the computer system functions as an apparatus for estimating heat treatment condition.

The CPU 20 stores a treatment condition library, relational expressions, and the like. The relational expressions include the following relational expressions:

(I) Relational expression indicative of correlation among desired nucleotide length, minimum nucleotide length, nucleotide length of nucleic acid before being subjected to the heat treatment, energy for fragmentation of nucleic acid in the heat treatment, heating temperature, and heat treatment start temperature; and
(II) Relational expression indicative of correlation among desired nucleotide length, minimum nucleotide length, nucleotide length of nucleic acid before being subjected to the heat treatment, speed of fragmentation of nucleic acid in the heat treatment, and heating time.

The relational expression of (I) above is expressed as Expression (I):

$$M = A+B\times\exp\{-H(T-D)\} \qquad (I)$$

(wherein, M represents a desired nucleotide length, A represents a minimum nucleotide length, B represents a nucleotide length of nucleic acid before being subjected to the heat treatment, H represents a constant of energy for fragmentation of nucleic acid in the heat treatment, T represents a heating temperature, and D represents a start temperature of the heat treatment). The relational expression of (II) above is expressed as Expression (II):

$$M = A+B\times\exp\{-Kt\} \qquad (II)$$

(wherein, M represents a desired nucleotide length, A represents a minimum nucleotide length, B represents a nucleotide length of nucleic acid before being subjected to the heat treatment, K represents a constant of speed of fragmentation of nucleic acid in the heat treatment, and t represents a heating time).

By using these relational expressions, a heat treatment condition for performing fragmentation of nucleic acid through heat treatment can be estimated in a simple manner.

The CPU 20 obtains a calculation result on the basis of information obtained through the input device 2b and the relational expressions stored in the hard disk 23. In addition, the CPU 20 estimates a heat treatment condition on the basis of the calculation result, and if necessary, also on the basis of the treatment condition library stored in the hard disk 23.

The ROM 21 is implemented by a mask ROM, a PROM, an EPROM, an EEPROM, or the like. The ROM 21 has stored therein computer programs to be executed by the CPU 20 and data used for the computer programs.

The RAM 22 is implemented by an SRAM, a DRAM, or the like. The RAM 22 is used for reading computer programs stored in the ROM 21 and in the hard disk 23. The RAM 22 is also used as a work area for the CPU 20 when the CPU 20 executes these computer programs.

The hard disk 23 has installed therein computer programs such as an operating system, an application program (a computer program for estimating a heat treatment condition), and the like that are to be executed by the CPU 20, and data to be used in execution of such computer programs.

The readout device 25 is implemented by a flexible disk drive, a CD-ROM drive, a DVD-ROM drive, and the like. The readout device 25 can read out computer programs or data stored in a portable storage medium 40.

The input/output interface 24 is implemented by, for example, a serial interface such as USB, IEEE1394, and RS-232C, a parallel interface such as SCSI, IDE, and IEEE1284, and an analog interface such as a D/A converter and an A/D converter. To the input/output interface 24, the input device 2b such as a keyboard and a mouse is connected. By using the input device 2b, an operator can input data to the computer body 2a.

The communication interface 26 is an Ethernet (registered trademark) interface, for example. The estimation apparatus 2 outputs an estimation result and the like obtained by the CPU 20, through the communication interface 26 to at least one of the monitor 2c and the heating apparatus 3. The estimation apparatus 2 can transmit print data to a printer through the communication interface 26.

The image output interface 27 is connected to the monitor 2c implemented by an LCD, a CRT, or the like.

Accordingly, the monitor 2c can output a video signal corresponding to image data provided by the CPU 20. The monitor 2c displays an image (screen) in accordance with the inputted video signal. The monitor 2c displays an estimation result and the like obtained by the CPU 20.

[Outline of process procedure performed by nucleic acid fragmentation system]

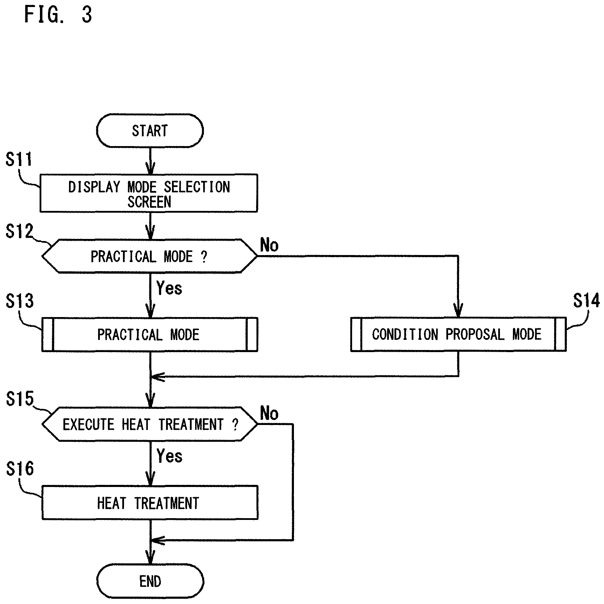

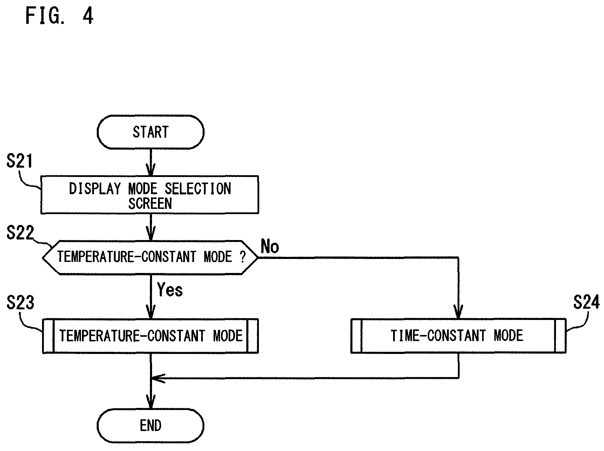

Next, with reference to FIGS. 3 and 4, the outline of the process procedure performed by the nucleic acid fragmentation system 1 will be described.

As shown in FIG 3, first, in step S 11, the CPU 20 of the estimation apparatus 2 causes, via the image output interface 27, the monitor 2c to display a mode selection screen. The mode selection screen is a screen for urging a user to select either one of a practical mode and a condition proposal mode.

The "practical mode" is a mode in which the user causes the nucleic acid fragmentation system 1 to execute heat treatment on the basis of a heat treatment condition set in advance by the user, thereby to actually perform nucleic acid fragmentation. In the practical mode, the nucleic acid fragmentation system 1 provides the user with information, advice, or the like that is useful for the heat treatment condition set in advance by the user. In the practical mode, the user can adopt as necessary the information or the advice provided by the nucleic acid fragmentation system 1, in consideration of the user's intention, knowledge, and the like. An assumed user of the practical mode is, for example, an expert well who is versed in nucleic acid fragmentation through heat treatment.

The "condition proposal mode" is a mode for causing the nucleic acid fragmentation system 1 to perform preparatory search and propose a heat treatment condition and the like for obtaining nucleic acid fragments having a desired average nucleotide length. Specifically, "the condition proposal mode" is a mode in which the user constructs a heat treatment condition and the like on site, following the information or advice provided by the nucleic acid fragmentation system 1. An assumed user of the condition proposal mode is, for example, a beginner and the like who are not versed in nucleic acid fragmentation through heat treatment. In the condition proposal mode, if the user accepts the proposed condition, the user can cause the nucleic acid fragmentation system 1 to execute heat treatment as appropriate.

In step S11, by operating the input device 2b, the user can designate a desired mode from the modes shown on the mode selection screen. In this specification, the term "nucleotide length" includes both concepts of the length of single-stranded nucleic acid and the length of double-stranded nucleic acid. The length of double-stranded nucleic acid is usually expressed in "bp" or "kb".

In step S12, the CPU 20 determines whether the mode selected by the user is the practical mode. When determining that the practical mode has been selected (Yes), the CPU 20 advances the process to step S 13. When determining that the practical mode has not been selected (No), the CPU 20 advances the process to step S14.

In step S 13, the CPU 20 performs a process for the practical mode. In the practical mode, the CPU 20 performs a process for a temperature-constant mode or a time-constant mode, in accordance with the process procedure shown in FIG. 4. In step S21 shown in FIG. 4, the CPU 20 causes, via the image output interface 27, the monitor 2c to display the mode selection screen. The mode selection screen is a screen for urging the user to select either one of the temperature-constant mode and the time-constant mode. Here, "the temperature-constant mode" in the practical mode is a mode for performing heat treatment at a heating temperature set in advance by the user. The "time-constant mode" in the practical mode is a mode for performing heat treatment for a heating time set in advance by the user. In step S 13, by operating the input device 2b, the user can designate a desired mode from the modes shown on the mode selection screen.

Next, in step S22, the CPU 20 determines whether the mode selected by the user is the temperature-constant mode. When determining that the temperature-constant mode has been selected (Yes), the CPU 20 advances the process to step S23. When determining that the temperature-constant mode has not been selected (No), the CPU 20 advances the process to step S24.

In step S23, the CPU 20 performs a process for the temperature-constant mode described later. In step S24, the CPU 20 performs a process for the time-constant mode described later.

Meanwhile, in step S 14, the CPU 20 performs a process for the condition proposal mode.

In the condition proposal mode, as in the practical mode, the CPU 20 performs a process for the temperature-constant mode or the time-constant mode in the process procedure shown in FIG. 4.

The "temperature-constant mode" in the condition proposal mode is a mode for causing the estimation apparatus 2 to propose a heat treatment condition for performing heat treatment at the heating temperature set in advance by the user. The "time-constant mode" in the condition proposal mode is a mode for causing the estimation apparatus 2 to propose a heat treatment condition for performing heat treatment in the heating time set in advance by the user.

After the process in step S13 or S14, the CPU 20 determines, in step S15, whether execution of heat treatment has been designated by the user. When determining that execution of heat treatment has been designated (Yes), the CPU 20 advances the process to step S16. When determining that execution of heat treatment has not been designated (No), the CPU 20 ends the process.

In step S16, the CPU 20 outputs, via the communication interface 26, a heat treatment condition and the designation of execution of heat treatment and to the heating apparatus 3. The heating apparatus 3 performs heat treatment in accordance with the heat treatment condition received from the CPU 20.

[Process procedure of practical mode]

(1) Process procedure of temperature-constant mode

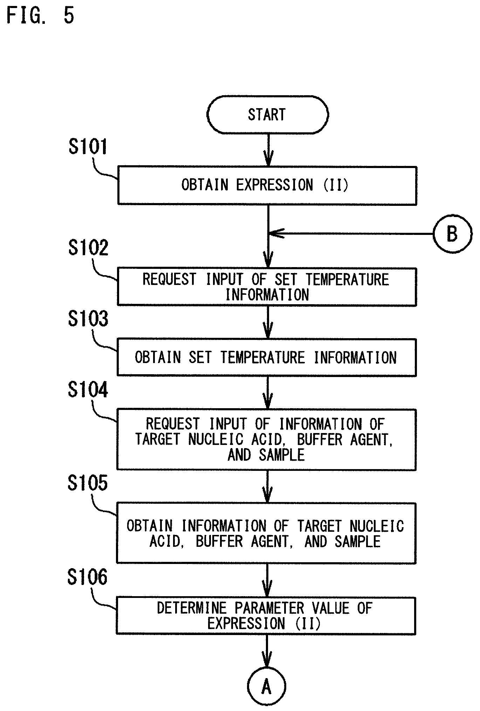

Next, with reference to FIG. 5 to FIG. 9, the process procedure of the temperature-constant mode in the practical mode performed by the estimation apparatus 2 will be described.

As shown in FIG. 5, first, in step S101, the CPU 20 obtains Expression (II) from the hard disk 23.

Next, in step S102, the CPU 20 requests the user to input set temperature information that indicates the set temperature to be used in the heat treatment. Specifically, the CPU 20 causes, via the image output interface 27, the monitor 2c to display a screen for urging the user to input the set temperature information. In step S102, by operating the input device 2b, the user can input the set temperature information.

Next, in step S103, the CPU 20 obtains the set temperature information inputted through the input device 2b. The CPU 20 can transmit, as necessary, the obtained set temperature information to the hard disk 23 to be temporarily stored therein.

Next, in step S104, the CPU 20 requests the user to input information of the target nucleic acid, information of the buffer agent, and information of the sample. Specifically, the CPU 20 causes, via the image output interface 27, the monitor 2c to display a screen for urging the user to input information of the target nucleic acid, information of the buffer agent, and information of the sample. In step S104, by operating the input device 2b, the user can input information of the target nucleic acid, information of the buffer agent, and information of the sample. Specifically, as the information of the target nucleic acid, the user can input information of the kind of the target nucleic acid. As the information of the buffer agent, the user can input information of the kind of the buffer agent contained in the sample. Further, as the information of the sample, the user can input information of the electric conductivity of the sample. Here, the target nucleic acid is DNA or RNA. Examples of the information of the kind of the buffer agent include information of components of the buffer agent. Examples of the components of the buffer agent include N-(2-Acetamido)iminodiacetic acid (hereinafter, also referred to as "ADA"), phosphoric acid, tris(hydroxymethyl)aminomethane (hereinafter, also referred to as "tris".

Next, in step S105, the CPU 20 obtains the information of the target nucleic acid, the information of the buffer agent, and the information of the sample, which have been inputted through the input device 2b. The CPU 20 can transmit, as necessary, the information of the target nucleic acid, the information of the buffer agent, and the information of the sample which have been obtained, to the hard disk 23 to be temporarily stored therein.

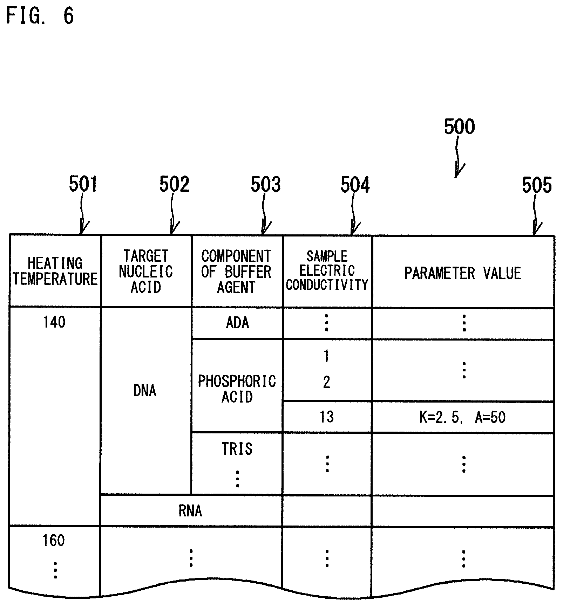

Next, in step S106, the CPU 20 determines parameter values of Expression (II). Specifically, the CPU 20 obtains a treatment condition library 500 shown in FIG 6 from the hard disk 23. Next, the CPU 20 determines the parameter values of Expression (II) on the basis of the set temperature information, the information of the target nucleic acid, the information of the buffer agent, and the information of the sample, and on the basis of the treatment condition library 500.

As shown in FIG 6, the treatment condition library 500 includes heating temperature information 501, target nucleic acid information 502, buffer agent information 503, sample electric conductivity information 504, and parameter value information 505 corresponding to these. The heating temperature information 501, the target nucleic acid information 502, the buffer agent information 503, the sample electric conductivity information 504, and the parameter value information 505 are information determined in advance through experiments and the like. These pieces of information determined in advance can be updated through input of information by the user, through electromagnetic provision of information, and the like. Examples of the manner of electromagnetic provision of information include delivery of information through an Internet website, in the form of attachment to a mail, and the like. The sample electric conductivity information 504 may be information obtained by converting the value of electric conductivity of the sample into another physical quantity such as the electric resistivity of the sample, the salt concentration of the sample, and the ionic strength of the sample. When the electric conductivity of the sample is expressed in terms of another physical quantity, the user may select such a physical quantity as appropriate depending on convenience.

In the treatment condition library 500 shown in FIG 6, the heat treatment conditions and the parameter values are hierarchically systematized in the order of the heating temperature information 501, the target nucleic acid information 502, the buffer agent information 503, the sample electric conductivity information 504, and the parameter value information 505. In this case, first, the CPU 20 can roughly narrow the heat treatment condition and the parameter values, on the basis of the set temperature information. Next, the CPU 20 can further narrow the narrowed heat treatment condition and parameter values, on the basis of the target nucleic acid information 502, the buffer agent information 503, and the sample electric conductivity information 504. Accordingly, the CPU 20 can determine parameter values of Expression (II). Examples of the parameter values determined in step S106 include the minimum nucleotide length A, the constant K of speed of fragmentation of nucleic acid in the heat treatment, and the like.

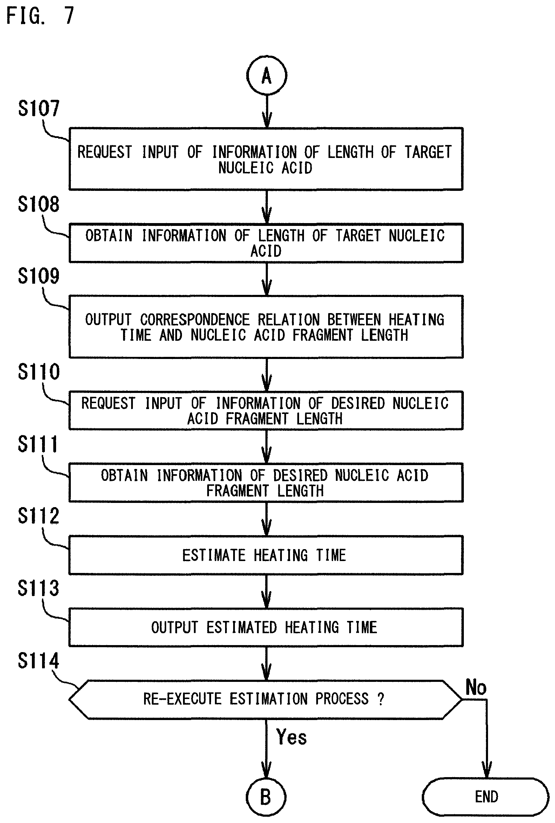

Next, in step S107 shown in FIG. 7, the CPU 20 requests the user to input information of the length of the target nucleic acid. Specifically, the CPU 20 causes, via the image output interface 27, the monitor 2c to display a screen for urging the user to input information of the length of the target nucleic acid. In step S107, by operating the input device 2b, the user can input information of the length of the target nucleic acid. Here, the "length of the target nucleic acid" corresponds to the "nucleotide length B of nucleic acid before being subjected to the heat treatment" in Expression (II).

Next, in step S108, the CPU 20 obtains the information of the length of the target nucleic acid inputted through the input device 2b. The CPU 20 can transmit, as necessary, the obtained information of the length of the target nucleic acid, to the hard disk 23 to be temporarily stored therein.

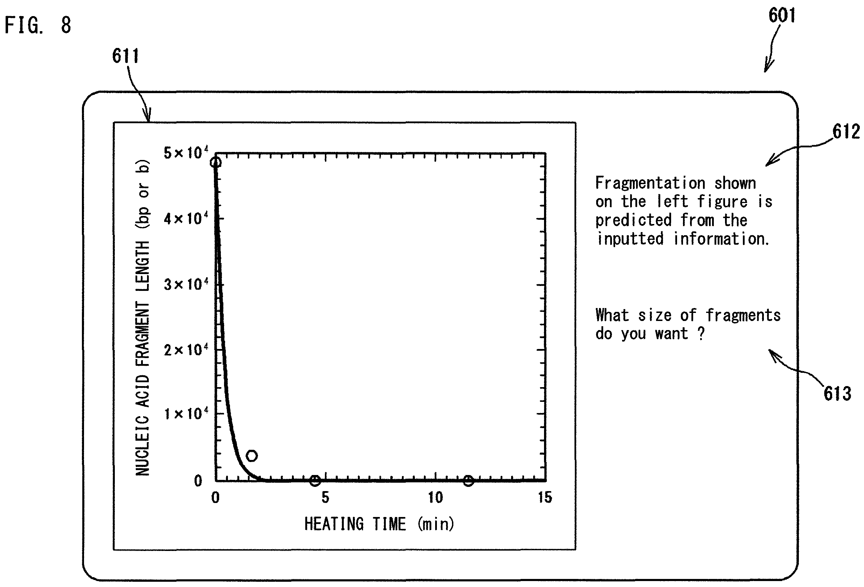

Next, in step S109, the CPU 20 outputs correspondence relation (hereinafter, also referred to as "estimated correspondence relation") between a heating time and a nucleic acid fragment length estimated on the basis of the information of the length of the target nucleic acid, and the parameter values determined in step S106 and Expression (II). Then, in step S110, the CPU 20 requests the user to input a desired nucleic acid fragment length. Specifically, the CPU 20 causes, via the image output interface 27, the monitor 2c to display a screen 601 shown in FIG. 8, for example. The screen 601 shown in FIG. 8 includes a graph 611 showing the estimated correspondence relation and comments 612 and 613 for the user. The X axis of the graph 611 represents heating time, and the Y axis of the graph 611 represents nucleic acid fragment length. The comment 612 is a comment regarding the presented graph 611. The comment 613 is a comment for requesting the user to input a desired nucleic acid fragment length. Here, the "desired nucleic acid fragment length" corresponds to the "desired nucleotide length M" in Expression (II).

Next, in step S111, the CPU 20 obtains information of the desired nucleic acid fragment length inputted through the input device 2b. The CPU 20 can transmit, as necessary, the obtained information of the desired nucleic acid fragment length, to the hard disk 23 to be temporarily stored therein. In step S111, by operating the input device 2b, the user plots a data point at the position of the desired nucleic acid fragment length in the graph 611 on the screen 601. Accordingly, the user can input the information of the desired nucleic acid fragment length.

Next, in step S 112, the CPU 20 estimates a heating time on the basis of the information of the desired nucleic acid fragment length inputted in step S111, and on the basis of parameter values of Expression (II) determined in step S106 and Expression (II).

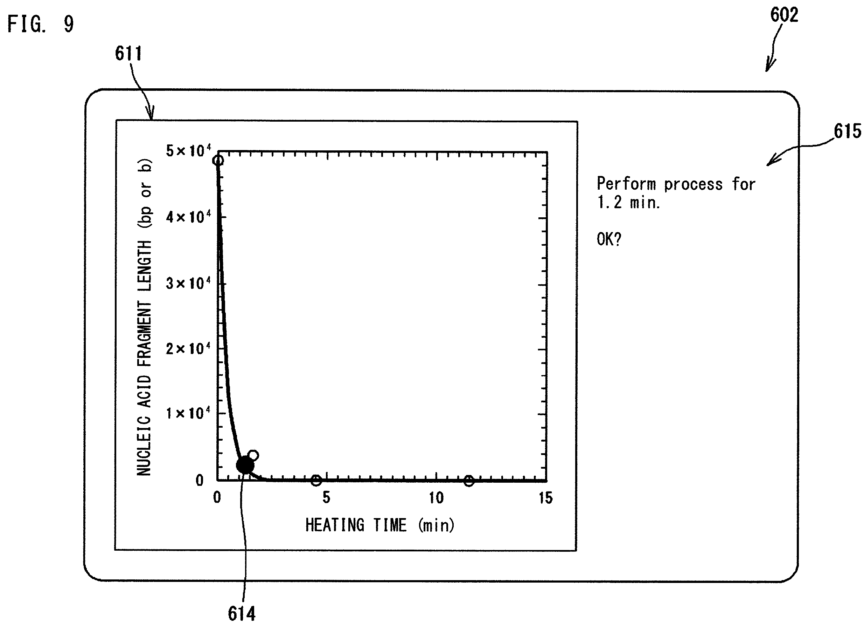

Next, in step S113, the CPU 20 outputs information of the heating time estimated in step S112. Specifically, the CPU 20 causes, via the image output interface 27, the monitor 2c to display a screen 602 shown in FIG 9. The screen 602 includes the graph 611 and a comment 615 for the user. In the screen 602, a data point 614 indicating the desired nucleic acid fragment length inputted by the user in step S111 is displayed on the graph 611. The comment 615 is a comment that presents the estimated heating time to the user. As shown in FIG. 9, the comment 615 may include wording for asking the user whether to perform heat treatment.

Next, in step S114 shown in FIG. 7, the CPU 20 determines whether the user requests re-execution of the estimation process. In step S 114, first, the CPU 20 causes, via the image output interface 27, the monitor 2c to display a screen for urging the user to input information regarding necessity/unnecessity of re-execution of the estimation process. Next, the CPU 20 obtains the information regarding necessity/unnecessity of re-execution of the estimation process inputted through the input device 2b. Then, on the basis of the inputted information, the CPU 20 determines whether the user requests re-execution of the estimation process. When determining that the user requests re-execution of the estimation process (Yes), the CPU 20 advances the process to step S102 shown in FIG. 5. When determining that the user does not request re-execution of the estimation process (No), the CPU 20 ends the process.

(2) Process procedure of time-constant mode

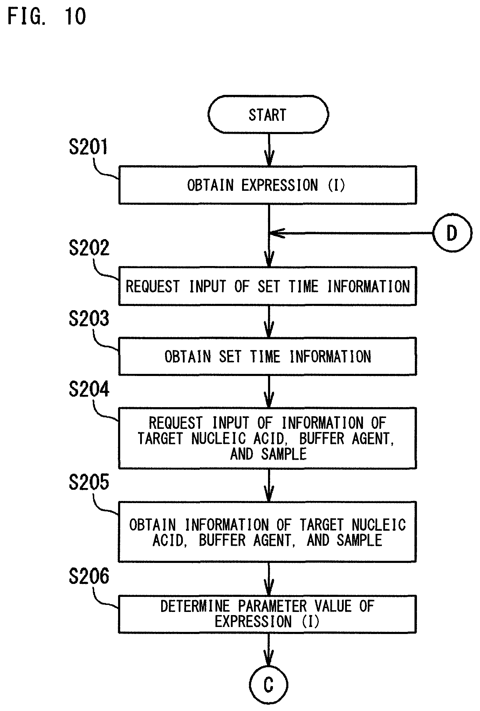

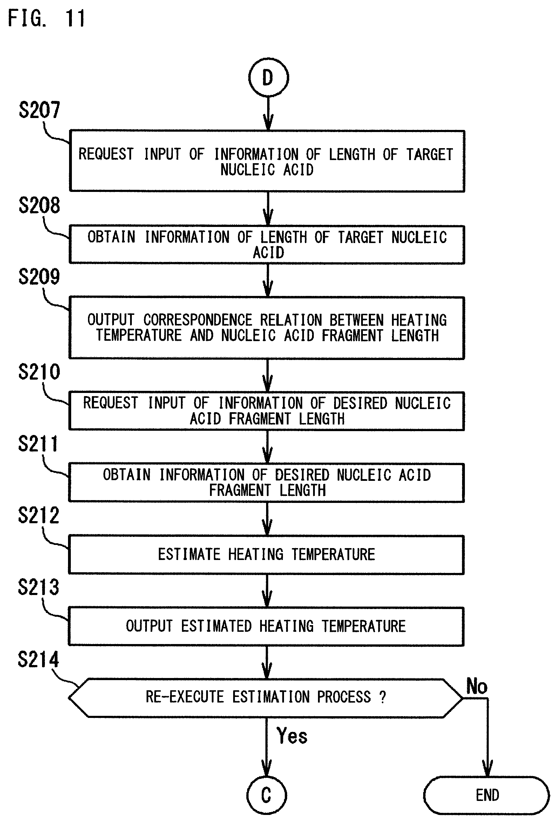

Next, with reference to FIG 10 and FIG. 11, the process procedure of the time-constant mode in the practical mode performed by the estimation apparatus 2 will be described. In the time-constant mode of the practical mode, the CPU 20 estimates a heating temperature on the basis of set time information provided by the user.

Except the following points, the process procedure of the time-constant mode in the practical mode is the same as the process procedure of the temperature-constant mode in the practical mode.

(A) In step S201 shown in FIG. 10, the CPU 20 obtains Expression (I).
(B) In step S202 shown in FIG 10, the CPU 20 requests the user to input information of set time to be used during the heat treatment.
(C) In step S203 shown in FIG. 10, the CPU 20 obtains set time information inputted through the input device 2b.
(D) In step S206 shown in FIG. 10, the CPU 20 determines parameter values of Expression (I).
(E) In step S209 shown in FIG 11, the CPU 20 outputs correspondence relation (estimated correspondence relation) between the heating temperature and the nucleic acid fragment length on the basis of information of the length of the target nucleic acid and on the basis of parameter values determined in step S206 in FIG. 10 and Expression (II).

(F) In step S212 shown in FIG. 11, the CPU 20 estimates a heating temperature on the basis of the information of the desired nucleic acid fragment length inputted in step S211, and on the basis of parameter values of Expression (I) determined in step S206 and Expression (I).

(G) In step S213 shown in FIG 11, the CPU 20 outputs information of the heating temperature estimated in step S212.

Examples of the parameter values determined in step S206 include the minimum nucleotide length A, the constant H of energy for fragmentation of nucleic acid in the heat treatment, and the like. The "length of the target nucleic acid" corresponds to the "nucleotide length B of nucleic acid before being subjected to the heat treatment" in Expression (I). The "desired nucleic acid fragment length" corresponds to the "desired nucleotide length M" in Expression (I).

[Modification of process procedure of practical mode]

In the process procedure of the practical mode shown in FIG 5 to FIG. 11, after the presentation of the estimated correspondence relation based on the set temperature information, the information of the target nucleic acid, the information of the buffer agent, the information of the sample, the information of the length of the target nucleic acid, and the like, the user is requested to input a desired nucleic acid fragment length. However, without presenting the estimated correspondence relation, the process of the practical mode may be advanced so as to request the user to input the desired nucleic acid fragment length.

[Process procedure of condition proposal mode]

(1) Process procedure of temperature-constant mode

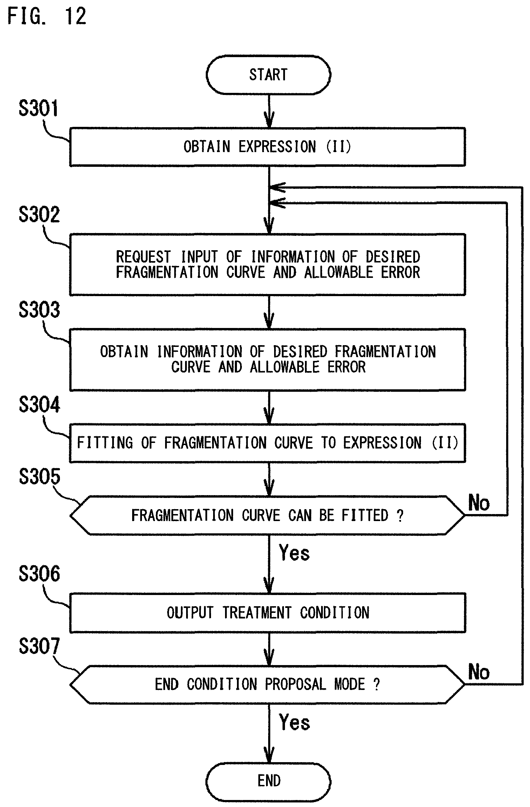

Next, with reference to FIG. 12 to FIG 16, the process procedure of the temperature-constant mode in the condition proposal mode performed by the estimation apparatus 2 will be described.

As shown in FIG. 12, first, in step S301, the CPU 20 obtains Expression (II) from the hard disk 23. Expression (II) is used as an expression of a regression line in the following steps.

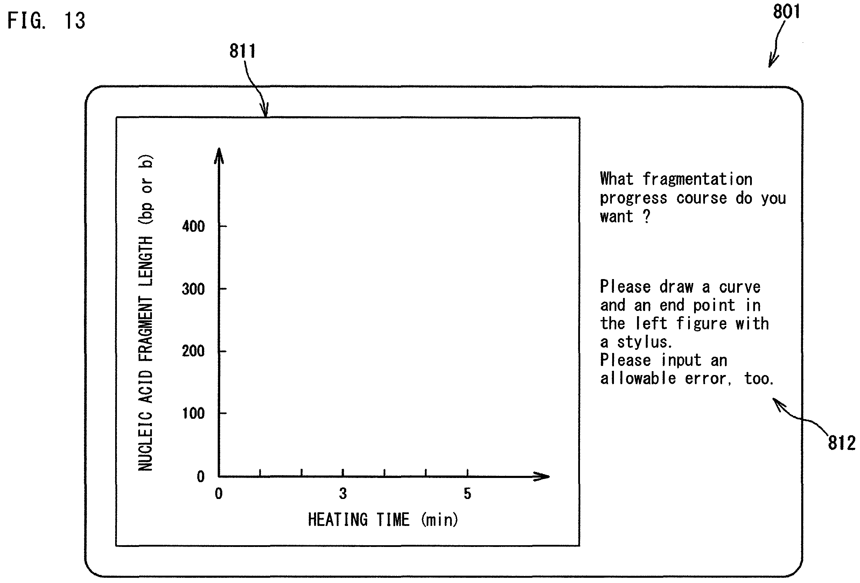

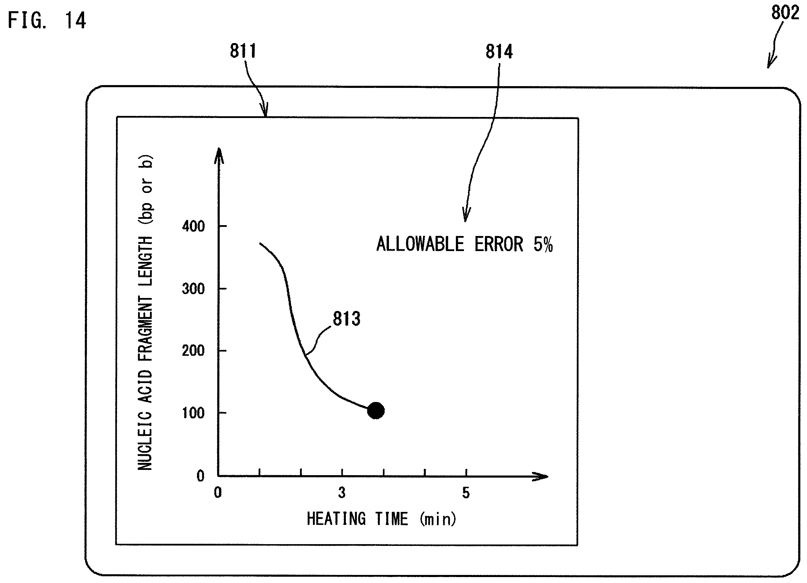

Next, in step S302, the CPU 20 requests the user to input information of a desired fragmentation curve and an allowable error. Specifically, the CPU 20 causes, via the image output interface 27, the monitor 2c to display a screen 801 for urging the user to input information of a desired fragmentation curve and an allowable error. As shown in FIG 13, the screen 801 includes a drawing area 811 and a comment 812. The drawing area 811 is the area for the user to draw a desired fragmentation curve therein. The X axis of the drawing area 811 represents heating time and the Y axis of the drawing area 811 represents nucleic acid fragment length. The comment 812 is a comment for urging the user to input information of a desired fragmentation curve and an allowable error. In step S302, as shown in FIG 14, by operating the input device 2b, the user can input a desired fragmentation curve 813 and allowable error information 814 in the drawing area 811 on a screen 802.

Next, in step S303 shown in FIG. 12, the CPU 20 obtains the information of the desired fragmentation curve and information of the allowable error which have been inputted through the input device 2b. The CPU 20 can transmit, as necessary, the information of the desired fragmentation curve and the information of the allowable error which have been obtained, to the hard disk 23 to be temporarily stored therein.

Next, in step S304, the CPU 20 performs fitting of the fragmentation curve obtained in step S303, with respect to Expression (II) in the range of the allowable error.

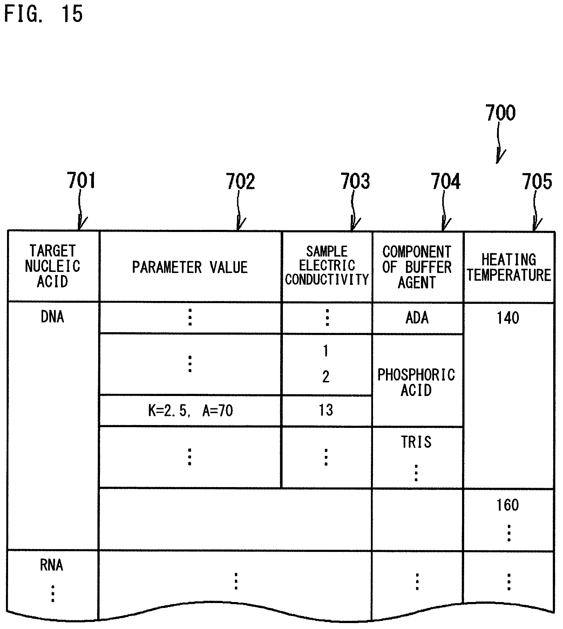

In step S304, prior to performing the fitting, if there is information not having been inputted in Expression (II), the lacking information is supplied by the CPU 20 from among heat treatment conditions and parameter values contained in the treatment condition library stored in the hard disk 23. When determining there is information not having been inputted in Expression (II), the CPU 20 obtains, from the hard disk 23, the lacking information contained in a treatment condition library 700 shown in FIG 15. As shown in FIG. 15, the treatment condition library 700 includes target nucleic acid information 701, parameter value information 702 of Expression (II), sample electric conductivity information 703, buffer agent information 704, and heating temperature information 705. The target nucleic acid information 701, the parameter value information 702, the sample electric conductivity information 703, the buffer agent information 704, and the heating temperature information 705 are information determined in advance through experiments and the like. The target nucleic acid information 701, the parameter value information 702, the sample electric conductivity information 703, the buffer agent information 704, and the heating temperature information 705 are the same as the target nucleic acid information 502, the parameter value information 505, the sample electric conductivity information 504, the buffer agent information 503, and the heating temperature information 501, respectively. In the treatment condition library 700 shown in FIG. 15, the heat treatment conditions and the parameter values are shown in two large groups so as to correspond to the respective kinds of the target nucleic acid.

The fitting is performed in accordance with the least-squares method or the like.

Next, in step S305 shown in FIG. 12, the CPU 20 determines whether the fragmentation curve obtained in step S303 can be fitted to Expression (II). In step S305, when values that are the same as or approximate to parameter values in the treatment condition library are obtained by fitting the fragmentation curve to Expression (II), it is possible to determine that the curve can be fitted. When determining that the fragmentation curve obtained in step S303 can be fitted to Expression (II) (Yes), the CPU 20 advances the process to step S306. In this case, the CPU 20 can estimate a heat treatment condition. Accordingly, a fragmentation curve indicating a heat treatment condition that satisfies Expression (II) can be obtained. When determining that the fragmentation curve obtained in step S303 cannot be fitted to Expression (II) (No), the CPU 20 advances the process to step S302. In this case, the CPU 20 requests again the user to input information of the desired fragmentation curve and the allowable error.

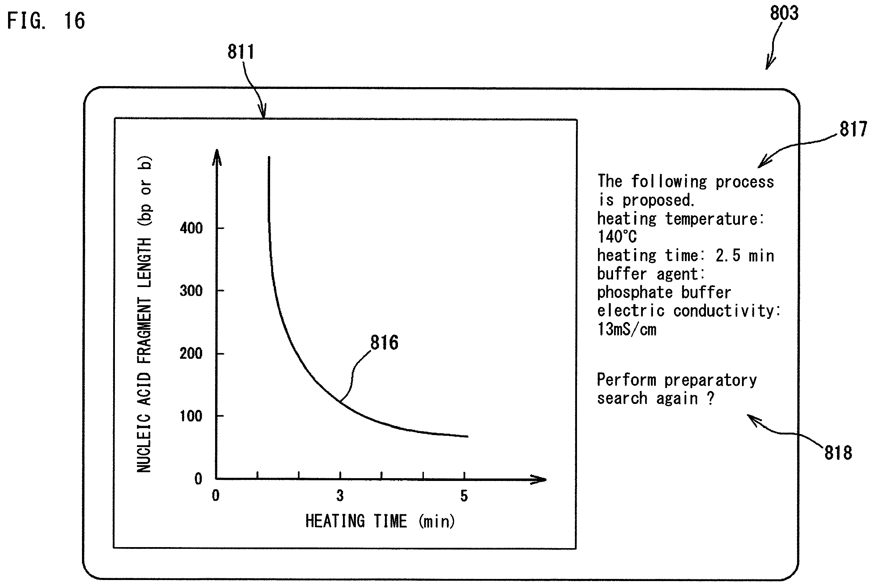

Next, in step S306, the CPU 20 outputs the heat treatment condition. Specifically, the CPU 20 causes, via the image output interface 27, the monitor 2c to display a screen 803 shown in FIG. 16. The screen 803 includes an estimated fragmentation curve 816 drawn in the drawing area 811, and comments 817 and 818 for the user. The estimated fragmentation curve 816 indicates the progress course of fragmentation based on the estimated heat treatment condition. The comment 817 is a comment for the user for presenting the estimated heat treatment condition. The comment 818 is a comment for asking the user whether to re-execute the temperature-constant mode in the condition proposal mode.

In step S306, the estimated heat treatment conditions can be ranked in terms of recommendation degree such that the heat treatment conditions having values closer to parameter values in the treatment condition library are ranked higher. In this case, it is possible to propose to the user heat treatment conditions having higher recommendation degrees, in the order of closer values to the parameter values.

Next, in step S307, the CPU 20 determines whether the user requests re-execution of the condition proposal mode. In step S307, first, the CPU 20 causes, via the image output interface 27, the monitor 2c to display a screen for urging the user to input information regarding necessity/unnecessity of re-execution of the condition proposal mode. Next, the CPU 20 obtains the information regarding necessity/unnecessity of re-execution inputted through the input device 2b. Then, on the basis of the inputted information, the CPU 20 determines whether the user requests re-execution of the condition proposal mode. When determining that the user requests re-execution of the condition proposal mode (Yes), the CPU 20 advances the process to step S302. When determining that the user does not request re-execution of the condition proposal mode (No), the CPU 20 ends the process.

(2) Process procedure of time-constant mode

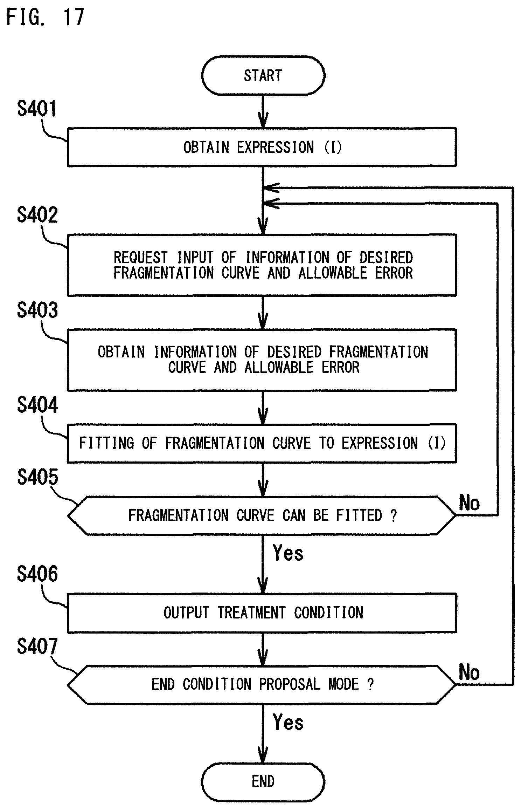

Next, with reference to FIG. 17, the process procedure of the time-constant mode in the condition proposal mode performed by the estimation apparatus 2 will be described.

Except the following points, the process procedure of the time-constant mode in the condition proposal mode is the same as the process procedure of the temperature-constant mode in the condition proposal mode.

(a) In step S401 shown in FIG. 17, the CPU 20 obtains Expression (I).
(b) In step S404 shown in FIG. 17, the CPU 20 performs fitting of the fragmentation curve obtained in step S403 with respect to Expression (I).
(c) In step S405 shown in FIG. 17, the CPU 20 determines whether the fragmentation curve obtained in step S403 shown in FIG 17 can be fitted to Expression (I).

[Modification]

Similarly to the above, it is also possible to adopt a mode in which any one or a plurality of conditions from among the heat treatment conditions are set to be constant. In this case, the estimation apparatus 2 can output, as an estimation result, heat treatment conditions not having been set to be constant.

[Example]

In the following description, each abbreviation has the following meaning.

<Abbreviation>

PBS: phosphate buffered saline [composition: 10 mM sodium phosphate buffer solution (pH7.8) and 150 mM sodium chloride]
10×PBS: 10-fold concentrated phosphate buffered saline

1×PBS: 1-fold concentrated phosphate buffered saline [composition: 10 mM sodium phosphate buffer solution (pH7.8) and 150 mM sodium chloride]

PB: potassium phosphate buffer solution [composition: 62 mM dipotassium hydrogenphosphate and 38 mM potassium dihydrogenphosphate, pH7.0]

TE: buffer solution having a composition of 10 mM tris and 1 mM ethylenediaminetetraacetic acid, pH7.5

STE: buffer solution having a composition of 10 mM tris, 1 mM ethylenediaminetetraacetic acid, and 50 mM sodium chloride, pH7.5

(Example 1)

(1) λDNA fragmentation process through heat treatment at various heating temperatures

A 10-fold concentrated phosphate buffered saline (10×PBS) (manufactured by Bio-Rad Laboratories, Inc.) was diluted with sterile water to obtain a 1-fold concentrated PBS (1 ×PBS). 1500 μL of the PBS and 50 μL of λDNA (manufactured by Takara Bio Inc., 0.3 μg/μL) were mixed together in a hydrothermal processing glass container. From the obtained mixture, 100 μL was each taken as an untreated sample (λDNA-containing sample at 20°C). Next, the hydrothermal processing glass container having the mixture therein was set in a microwave synthesis reaction apparatus (product name: MultiSYNTH, manufactured by Milestone General K.K.). Then, heat treatment was performed for 10 seconds at the heating temperature of 120°C, 140°C, 160°C, or 180°C, to obtain a sample. The thermal profile of the heat treatment was set as follows.

<Thermal profile>

Steps of (i-1) to (i-4) below:

(i-1) raising temperature from ordinary temperature (20°C) to 100°C in 30 seconds;
(i-2) raising temperature from 100°C to a predetermined heating temperature in 60 seconds;
(i-3) heating for 10 seconds at the predetermined heating temperature; and
(i-4) cooling at 20°C.

(2) Evaluation of λDNA fragmentation through heat treatment

3μL of a buffer solution for electrophoresis (product name: ×6 Loading buffer, manufactured by Takara Bio Inc.) was added to 15μL of the sample obtained in (1) of Example 1, to obtain an electrophoresis sample. By using an electrophoresis apparatus (product name: vertical mini electrophoresis system, manufactured by Invitrogen), an electrophoresis gel (product name: 6% TBE GEL, 1.0 mm, 12 wells, manufactured by Invitrogen), and a running buffer (1-fold concentrated TBE (1×TBE)), electrophoresis was performed for each electrophoresis sample and a marker for 23 minutes under a voltage of 200 V. As the marker, Wide-Range DNA Ladder (50-10000 bp) (product name, manufactured by Takara Bio Inc.) and λ-Hind III digest (product name, manufactured by Takara Bio Inc.) were used. The 1 ×TBE was prepared by 10-fold diluting a 10-fold concentrated nucleic acid electrophoresis premix buffer (product name: 10×TBE, manufactured by Bio-Rad Laboratories, Inc.).

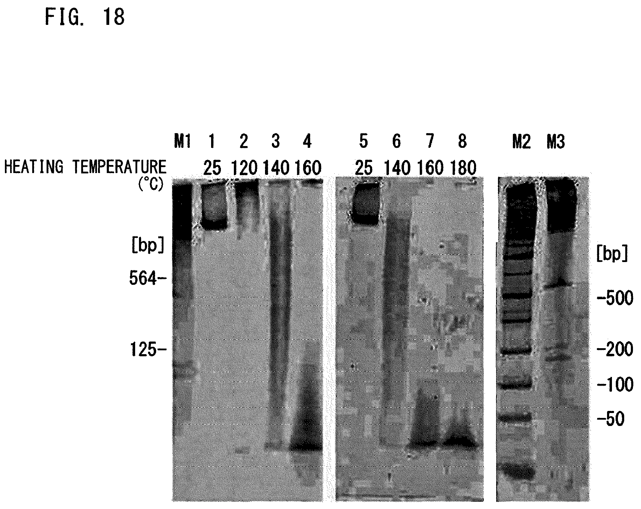

The obtained electrophoresis gel was immersed to be stained in a nucleic acid stain (diluted solution obtained by 10000-fold diluting Gel Star (product name, manufactured by Lonza) with 1 ×TBE) for 30 minutes. The stained electrophoresis gel was subjected to an image analysis system (product name: Personal Molecular Imager, manufactured by Bio-Rad Laboratories, Inc.), to obtain an image at G excitation. The results are shown in FIG. 18. In FIG. 18, lanes M1 and M3 each show an electrophoresis pattern of a marker (product name: λ-Hind III digest, manufactured by Takara Bio Inc.), lane M2 shows an electrophoresis pattern of a marker (product name: Wide-Range DNA Ladder (50-10000 bp), manufactured by Takara Bio Inc.), lanes 1 and 5 each show an electrophoresis pattern of the untreated sample, lane 2 shows an electrophoresis pattern of the sample having been subjected to the heat treatment at 120°C, lanes 3 and 6 each show an electrophoresis pattern of the sample having been subjected to the heat treatment at 140°C, lanes 4 and 7 each show an electrophoresis pattern of the sample having been subjected to the heat treatment at 160°C, and lane 8 shows an electrophoresis pattern of the sample having been subjected to the heat treatment at 180°C.

From the results shown in FIG 18, it was found that the higher the heating temperature during the heat treatment, the shorter the average length of the obtained fragments.

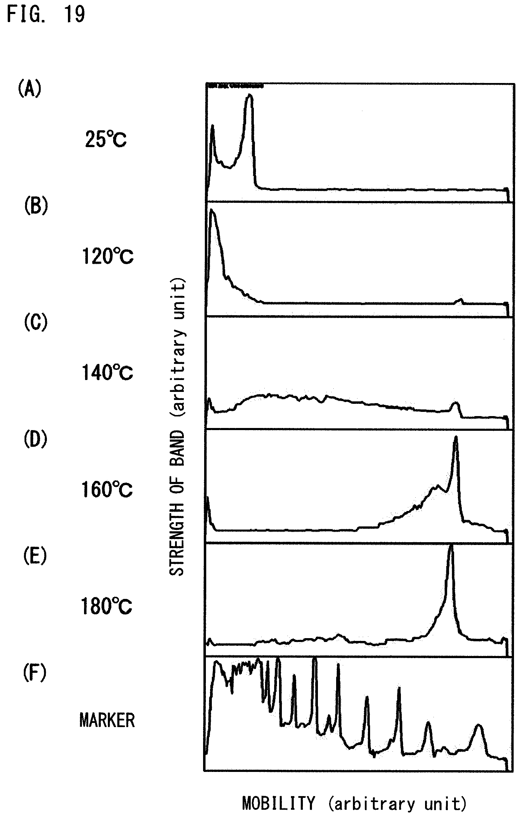

A gel densitometry function of image processing software (product name: Image J, provided by National Institutes of Health, USA) was applied to the image data shown in FIG. 18. Then, a mobility spectrum showing the relationship between the magnitude of the density of the band in each lane and the mobility of the band was obtained. The results are shown in FIG. 19. In FIG 19, (A) shows the mobility spectrum of the untreated sample, (B) shows the mobility spectrum of the sample having been subjected to the heat treatment at 120°C, (C) shows the mobility spectrum of the sample having been subjected to the heat treatment at 140°C, (D) shows the mobility spectrum of the sample having been subjected to the heat treatment at 160°C, (E) shows the mobility spectrum of the sample having been subjected to the heat treatment at 180°C, and (F) shows the mobility spectrum of the marker. Each mobility shown in FIG. 19 is a value obtained by measuring and normalizing the distance by which the band in the electrophoresis gel moved.

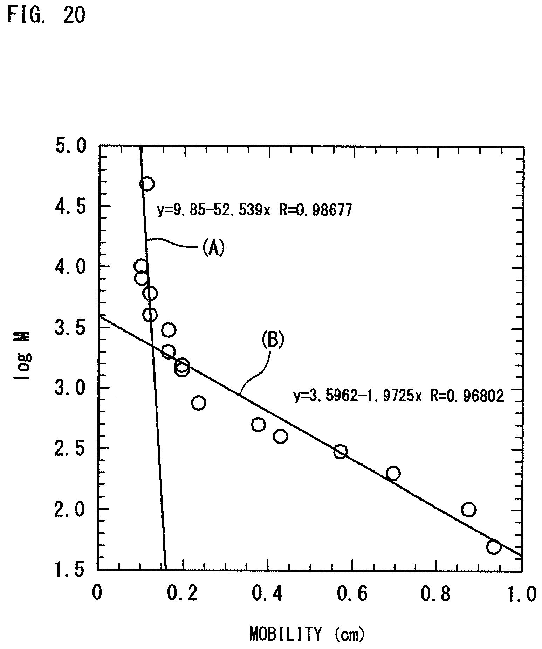

Further, from the mobility spectrum of the marker shown in (F) of FIG. 19, a nucleic acid fragment length M and the mobility of the marker were obtained. From the nucleic acid fragment length M, logM was obtained. "LogM" is a logarithm of the nucleic acid fragment length M having 10 as the base. By performing plotting on two-dimensional coordinates whose X axis represented the mobility of the band of nucleic acid fragments in the marker and whose Y axis represented logM, a calibration curve shown in FIG. 20 was obtained.

The group of data points derived from the marker on the calibration curve shown in FIG. 20 was classified into a group of data points in a high molecular weight area and a group of data points in a low molecular weight area.

<Classification criteria>

Group of data points in the high molecular weight area: group of data points whose nucleic acid fragment length is not less than 30000 bp
Group of data points in the low molecular weight area: group of data points whose nucleic acid fragment length is less than 30000 bp

With respect to each of the group of data points in the high molecular weight area and the group of data points in the low molecular weight area, an approximate straight line was obtained by using data analysis/graph creation software (product name: KaleidaGraph, manufactured by Hulinks).

As a result, it was found that an approximate straight line (A) shown in FIG. 20 was a straight line (R value = 0.98677) represented by Expression (III):

$$logM = 9.85\text{-}52.539\times\mu \qquad (III)$$

(wherein, M represents nucleic acid fragment length, and $\mu$ represents mobility). In addition, an approximate straight line (B) shown in FIG. 20 was a straight line (correlation coefficient R value = 0.96802) represented by Expression (IV):

$$logM=3.5962\text{-}1.9725\times\mu \qquad (IV)$$

It was considered that each coefficient in Expressions (III) and (IV) would vary depending on the hardness, the size, and the like of the electrophoresis gel that was used. Thus, Expressions (III) and (IV) were generalized to obtain Expression (V):

$$logM = a\text{-}b\times\mu \qquad (V)$$

(wherein, M represents nucleic acid fragment length, $\mu$ represents mobility, and "a" and "b" each represent a number determined through curve fitting).

(3) Verification of relationship between nucleic acid fragment length and heating temperature during heat treatment

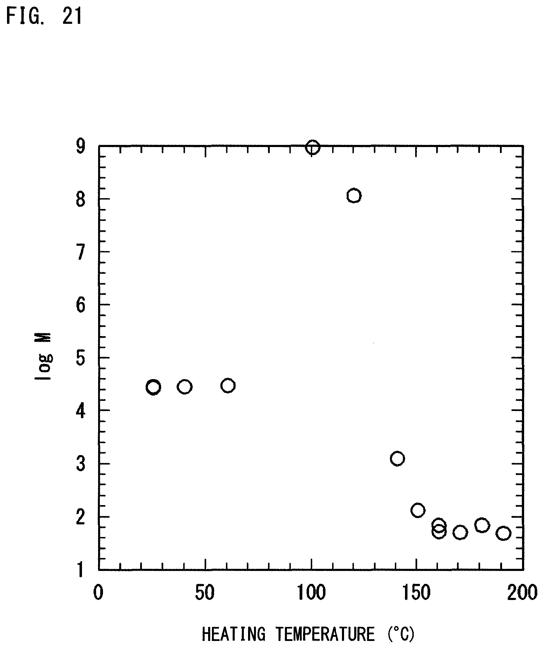

Except that the heating temperature was set at 40°C, 60°C, 100°C, 150°C, 170°C, or 190°C, the same operation was performed as that in (1) and (2) of Example 1, whereby the mobility spectrum at each temperature was obtained. It was found that nucleic acid aggregated when the heating temperature was near 100°C. Next, from among the peaks in the mobility spectrum at each temperature, the mobility of the band that showed the highest peak was obtained by measuring and normalizing the distance by which the band in the electrophoresis gel moved. By putting the obtained mobility into Expression (III) or Expression (IV), the nucleic acid fragment length M was calculated. From the nucleic acid fragment length M, logM was obtained. The heating temperature and logM were plotted on two-dimensional coordinates whose X axis represented heating temperature and whose Y axis represented logM. The result is shown in FIG. 21.

With reference to the result shown in FIG. 21, when the heating temperature was in a low temperature area of 20 to 60°C, no significant change was observed in the nucleic acid fragment length M. When the heating temperature was near 100°C, it was found that the value of logM had increased. Further, when the heating temperature was in a high temperature area of 130°C or higher, it was quantitatively confirmed that nucleic acid fragmentation had occurred in association with rise in temperature. It should be noted that when the heating temperature was near 100°C, increase in the value of logM was confirmed. In addition, when the heating temperature was near 100°C, aggregation of nucleic acid was observed. Therefore, it was speculated that the increase in the value of logM was caused by the aggregation of nucleic acid.

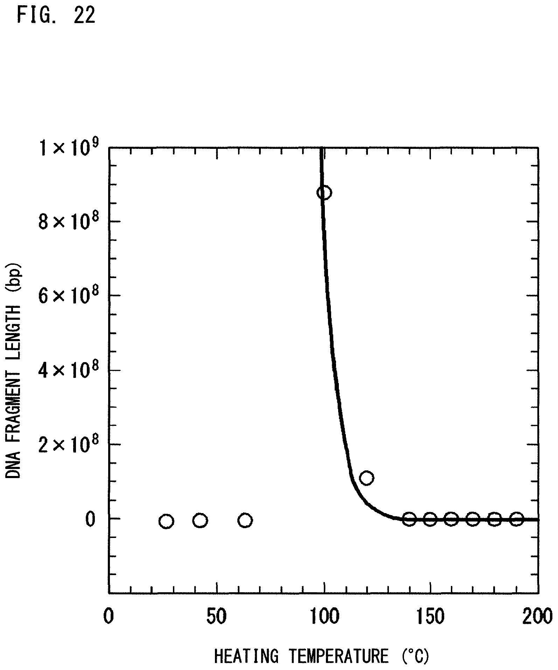

Next, from the data points on the graph shown in FIG. 21, data points at heating temperatures at which fragmentation occurred, and data points at heating temperatures at which fragmentation did not occur were extracted. By using the extracted data points, heating temperatures and nucleic acid fragment lengths were re-plotted on two-dimensional coordinates whose X axis represented temperature and whose Y axis represented nucleic acid fragment length. The result is shown in FIG. 22. It was speculated that nucleic acid heated at a heating temperature near 50°C was not fragmented. Thus, the nucleic acid fragment length M of nucleic acid heated at a heating temperature near 50°C was defined as 0. In FIG 22, in the group of the plotted data points, with respect to a group of data points having a heating temperature of 100°C or higher, an approximate curve was obtained by using data analysis/graph creation software (product name: KaleidaGraph, manufactured by Hulinks).

It was found that the approximate curve shown in FIG 22 was represented by Expression (I):

$$M = A+B\times\exp\{-H(T-D)\} \qquad (I)$$

(wherein, M represents nucleic acid fragment length (desired nucleotide length), A represents a minimum nucleotide length, B represents a nucleotide length of nucleic acid before being subjected to the heat treatment, H represents a constant of energy for fragmentation of nucleic acid in the heat treatment, T represents a heating temperature, and D represents a start temperature of the heat treatment). The approximate curve shown in FIG. 22 was a function of temperature T. Thus, the value of T which is a variable in the X axis was applied to Expression (I), whereby A, B, H, and D in Expression (I) were obtained. The results were as follows. A = 50, B = 7.94x108, H = 0.147, and D = 100. The obtained A, B, H, and D were values reflecting the heat treatment in which DNA having 79400 bp was heated for 10 seconds. Therefore, it was found that the heating temperature in the 10-second heat treatment can be obtained by putting a desired nucleotide length into Expression (I). Thus, it was found that, according to Expression (I), it is possible to determine a heating temperature that allows obtainment of a desired nucleotide length in a heat treatment performed for a certain heating time.

(Example 2)

Except that the kind of nucleic acid and the buffer solution were changed as in table 1, the same operation as in Example 1 was performed. Then, by using Expression (I), the constant of energy for fragmentation of nucleic acid in the heat treatment for each condition was calculated. The result is shown in table 1.

From the result shown in table 1, it was found that the value of the constant H of energy for fragmentation of nucleic acid in the heat treatment differs depending on the kind of the target nucleic acid, the kind of the buffer agent contained in the sample, and the electric conductivity of the sample.

[Table 1]

| Target nucleic acid | Component of buffer agent | Electric conductivity (mS/cm) | H |
|---|---|---|---|
| DNA | Phosphoric acid (PBS) | 13.3 | 0.15 |
| | Phosphoric acid (PB) | 8 | 0.06 |
| | Tris (TE) | 0.8 | 0.36 |
| RNA | Phosphoric acid (PBS) | 13.3 | 0.039 |

(Example 3)

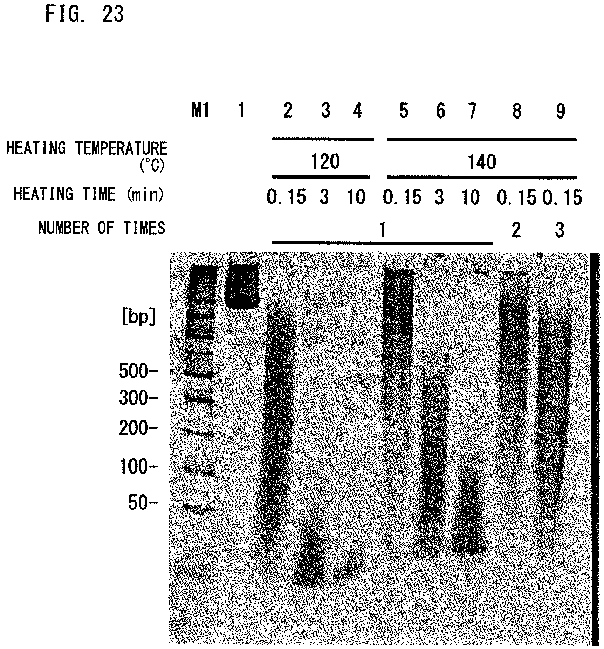

Except that the heating temperature was fixed at 140°C and the heating time was set to various times, the same operation as in (1) and (2) of Example 1 was performed, and an image of the electrophoresis gel at G excitation was obtained. The results are shown in FIG. 23. In FIG. 23, lane M1 shows an electrophoresis pattern of a marker (product name: λ-Hind III digest, manufactured by Takara Bio Inc.), lane 1 shows an electrophoresis pattern of the untreated sample, lane 2 shows an electrophoresis pattern of the sample having been subjected to the heat treatment at 120°C for 0.15 minutes, lane 3 shows an electrophoresis pattern of the sample having been subjected to the heat treatment at 120°C for 3 minutes, lane 4 shows an electrophoresis pattern of the sample having been subjected to the heat treatment at 120°C for 10 minutes, lanes 5, 8, and 9 each show an electrophoresis pattern of the sample having been subjected to the heat treatment at 140°C for 0.15 minutes, lane 6 shows an electrophoresis pattern of the sample having been subjected to the heat treatment at 140°C for 3 minutes, and lane 7 shows an electrophoresis pattern of the sample having been subjected to the heat treatment at 140°C for 10 minutes. The thermal profile of the heat treatment was set as follows. Here, the heating time means the heating time according to (ii-3) below. Cooling in (ii-4) was performed through heat dissipation from the sample, by blowing air to the sample.

<Thermal profile>

Steps of (ii-1) to (ii-4) below:

(ii-1) raising temperature from ordinary temperature (20°C) to 100°C in 30 seconds;
(ii-2) raising temperature from 100°C to 120°C or 140°C in 60 seconds;
(ii-3) heating at 140°C for 0.15 to 10 minutes; and
(ii-4) cooling from 120°C or 140°C to 20°C.

From the results shown in FIG. 23, it was found that the longer the heating time during the heat treatment, the shorter the average length of the obtained fragments.

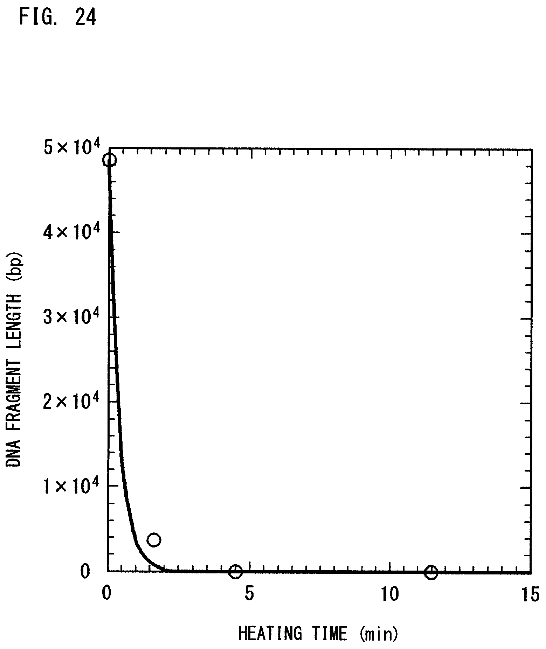

Among the results shown in FIG 23, by using the image processing software (product name: Image J, provided by National Institutes of Health, USA) to the electrophoresis patterns obtained at the heating temperature of 140°C, a mobility spectrum showing the relationship between the magnitude of the density of the band in each lane and the mobility of the band was obtained. Next, from among the peaks in the mobility spectrum for each heating time, the mobility of the band that showed the highest peak was calculated. With reference to the mobility spectrum of the marker, the relationship between the mobility of the band of nucleic acid fragments and the nucleic acid fragment length was examined. The heating time and the nucleic acid fragment length were plotted on two-dimensional coordinates whose X axis represented heating time and whose Y axis represented nucleic acid fragment length. Next, with respect to the group of the plotted data points, an approximate curve was obtained by using the data analysis/graph creation software (product name: KaleidaGraph, manufactured by Hulinks). The result is shown in FIG 24. In FIG. 24, the heating time was obtained by totaling the heating times in step (ii-1), step (ii-2), and step (ii-3) of the thermal profile.

It was found that the approximate curve shown in FIG 24 was represented by Expression (II):

$$M = A + B \times \exp\{-Kt\} \qquad (II)$$

(wherein, M represents nucleic acid fragment length (desired nucleotide length), A represents a minimum nucleotide length, B represents a nucleotide length of nucleic acid before being subjected to the heat treatment, K represents a constant of speed of fragmentation of nucleic acid in the heat treatment, and t represents a heating time). The approximate curve shown in FIG. 24 was a function of time t. Thus, the value of t was put into Expression (II), whereby A, B, and K in Expression (II) were obtained. The results were as follows. A = 50, B = $4.85 \times 10^4$, and K= 2.5. The obtained A, B, and K were values reflecting the heat treatment in which DNA having 48500 bp was heated at 140°C. Therefore, it was found that the heating time in the heat treatment at 140°C can be obtained by putting a desired nucleotide length into Expression (II). Thus, it was found that, according to Expression (II), it is possible to determine a heating time that allows obtainment of a desired nucleotide length in a heat treatment performed for a certain heating temperature.

(Example 4)

Except that the kind of nucleic acid and the buffer solution were changed as in table 2, the same operation as in Example 3 was performed. Then, by using Expression (II), the constant K of speed of fragmentation of nucleic acid in the heat treatment for each condition was calculated. The result is shown in table 2.

From the result shown in table 2, it was found that the value of the constant K of speed of fragmentation of nucleic acid in the heat treatment differs depending on the kind of the target nucleic acid, the kind of the buffer agent contained in the sample, and the electric conductivity of the sample.

[Table 2]

| Target nucleic acid | Temperature (°C) | Component of buffer agent | Electric conductivity (mS/cm) | K |
|---|---|---|---|---|
| DNA | 140 | Phosphoric acid (PBS) | 13.3 | 2.5 |
| | 160 | | 13.3 | 1.4 |
| | 180 | | 13.3 | 2.9 |
| | 200 | | 13.3 | 4 |
| | 180 | Tris (STE) | 5.2 | 5.5 |
| | 140 | Tris (TE) | 0.8 | 5.7 |
| RNA | 160 | Phosphoric acid (PBS) | 13.3 | 3.6 |

(Example 5)

(1) λDNA fragmentation process through heat treatment at various heating temperatures

25 μL of λDNA (manufactured by Takara Bio Inc., 0.3 μg/μL) was added to 1.5 mL of PB, to obtain a mixture. The obtained mixture was put in an autoclave (product name: science autoclave NCC-1701, manufactured by AS ONE Corp.), and heat treatment was performed at 95°C, 121°C, or 132°C, for 20 minutes.

(2) Evaluation of λDNA fragmentation through heat treatment

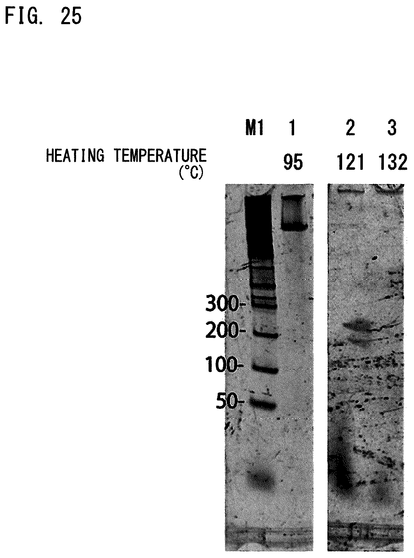

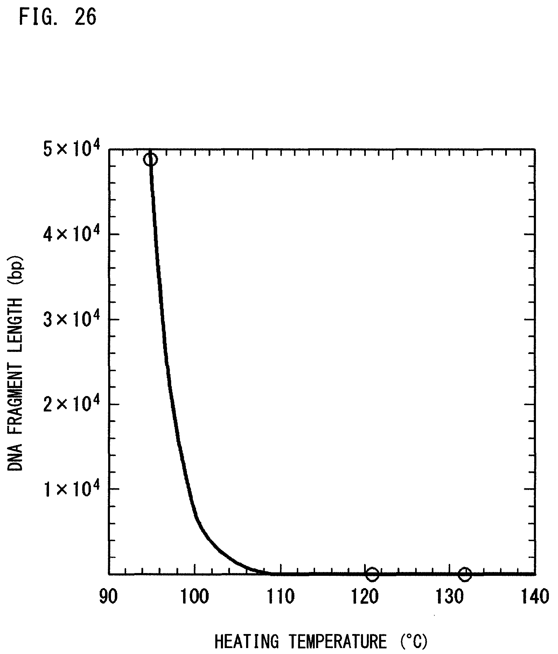

Except that 10 μL of the sample obtained in (1) of Example 5 was used, the same operation as in (2) of Example 1 performed to obtain an electrophoresis gel.
The obtained electrophoresis gel was immersed to be stained in a nucleic acid stain (diluted solution obtained by 10000-fold diluting SYBR Green II with 1×TBE) for 30 minutes. The electrophoresis gel having the stained nucleic acid was subjected to the image analysis system (product name: Personal Molecular Imager, manufactured by Bio-Rad Laboratories, Inc.), to obtain a fluorescence image. The results are shown in FIG 25. In FIG. 25, lane M1 shows an electrophoresis pattern of a marker (product name: λ-Hind III digest, manufactured by Takara Bio Inc.), lane 1 shows an electrophoresis pattern of the untreated sample, lane 2 shows an electrophoresis pattern of the sample having been subjected to the heat treatment at 95°C, lane 3 shows an electrophoresis pattern of the sample having been subjected to the heat treatment at 121 °C, and lane 4 shows an electrophoresis pattern of the sample having been subjected to the heat treatment at 132°C. In FIG. 25, the numerical values on the left in lane M1 respectively represent 300 bp, 200 bp, 100 bp, and 50 bp, from the top in order.
From the results shown in FIG 25, it was found that, even when heat treatment is performed according to a heat transfer method that uses an autoclave, the higher the heating temperature during the heat treatment, the shorter the average length of the obtained fragments.
By using FIG. 25 and the image processing software (product name: Image J, provided by National Institutes of Health, USA), a mobility spectrum showing the relationship between the magnitude of the density of the band in each lane and the mobility of the band was obtained. Next, from among the peaks in the mobility spectrum at each heating temperature, the mobility of the band that showed the highest peak was calculated. With reference to the mobility spectrum of the marker, the relationship between the mobility of the band of nucleic acid fragments and the nucleic acid fragment length was examined. The heating temperature and the nucleic acid fragment length were plotted on two-dimensional coordinates whose X axis represented heating temperature and whose Y axis represented nucleic acid fragment length. Next, fitting of the group of the plotted data points to Expression (I) was performed by using the data analysis/graph creation software (product name: KaleidaGraph, manufactured by Hulinks). The result is shown in FIG. 26.
The curve shown in FIG. 26 was a function of the temperature T shown in Expression (I). Thus, the value of T was put into Expression (I), whereby A, B, H, and D in Expression (I) were obtained. The results were as follows. A = 14.677, B = $5.41 \times 10^4$, H = 0.36, and D = 94.693. The obtained A, B, and D were values reflecting the heat treatment in which DNA having 48500 bp was heated for 20 minutes. Therefore, it was found that the heating temperature in the heat treatment for 20 minutes can be obtained by putting a desired nucleotide length into Expression (I). Thus, it was found that, according to Expression (I), it is possible to determine a heating temperature that allows obtainment of a desired nucleotide length in a heat treatment performed at a certain heating time, irrespective of what heating means is used.

(Example 6)

(1) λDNA fragmentation process through heat treatment for various heating times

25 μL of λDNA (manufactured by Takara Bio Inc., 0.3 μg/μL) was added to 1.5 mL of a phosphate buffer solution, to obtain a mixture. The obtained mixture was put in the autoclave (product name: science autoclave NCC-1701, manufactured by AS ONE Corp.), and heat treatment was performed at 132°C, for 5 minutes, 10 minutes, 15 minutes, or 30 minutes.

(2) Evaluation of λDNA fragmentation through heat treatment

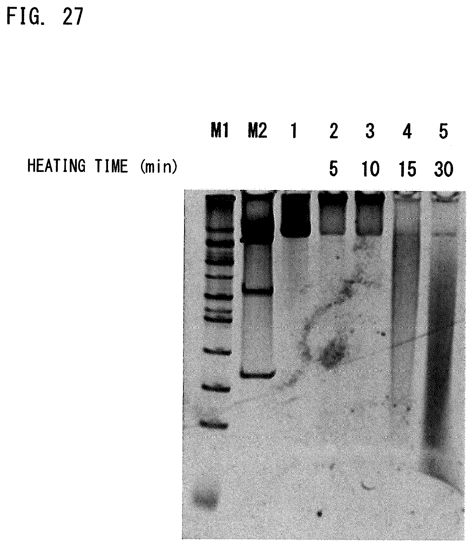

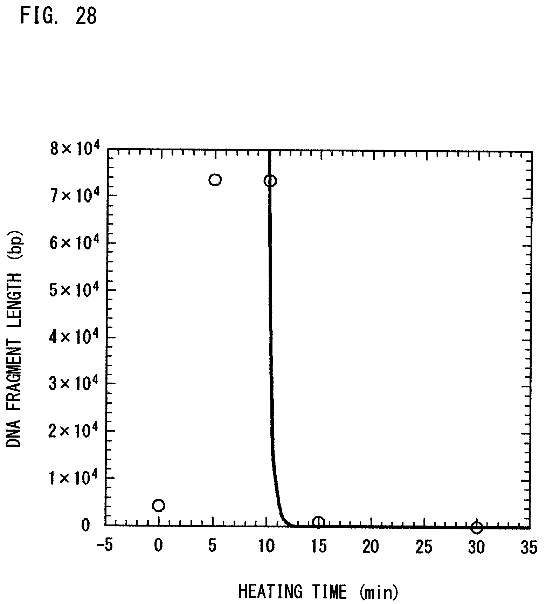

Except that 10 μL of the sample obtained in (1) of Example 6 was used, the same operation as in (2) of Example 5 was performed to obtain a fluorescence image of the electrophoresis gel. The results are shown in FIG 27. In FIG. 27, lane M1 shows an electrophoresis pattern of a marker (product name: Wide-Range DNA Ladder (50-10000 bp), manufactured by Takara Bio Inc.), lane M2 shows an electrophoresis pattern of a marker (product name: λ-Hind III digest, manufactured by Takara Bio Inc.), lane 1 shows an electrophoresis pattern of the untreated sample, lane 2 shows an electrophoresis pattern of the sample having been subjected to the heat treatment for 5 minutes, lane 3 shows an electrophoresis pattern of the sample having been subjected to the heat treatment for 10 minutes, lane 4 shows an electrophoresis pattern of the sample having been subjected to the heat treatment for 15 minutes, and lane 5 shows an electrophoresis pattern of the sample having been subjected to the heat treatment for 30 minutes.
From the results shown in FIG. 27, it was found that, even when the heat treatment was performed according to a heat transfer method that uses an autoclave, the longer the heating time of the heat treatment, the shorter the average nucleotide length of the obtained nucleic acid fragments.
By using FIG 27 and the image processing software (product name: Image J, provided by National Institutes of Health, USA), a mobility spectrum showing the relationship between the magnitude of the density of the band in each lane and the mobility of the band was obtained. Next, from among the peaks in the mobility spectrum for each heating time, the mobility of the band that showed the highest peak was calculated. With reference to the mobility spectrum of the marker, the relationship between the mobility of the band of nucleic acid fragments and the nucleic acid fragment length was examined. The heating time and the nucleic acid fragment length were plotted on two-dimensional coordinates whose X axis represented heating time and whose Y axis represented nucleic acid fragment length. Next, fitting of the group of the plotted data points to Expression (II) was performed by using the data analysis/graph creation software (product name: KaleidaGraph, manufactured by Hulinks). The result is shown in FIG 28.
The curve shown in FIG. 28 was a function of time t shown in Expression (II). Thus, the value of t was put into Expression (II), whereby A, B, and K in Expression (II) were obtained. The results were as follows. A = 78.5, B = 7.94 × 104, and K = 2.5. The obtained A, B, K were values reflecting the heat treatment in which DNA having 79400 bp were heated at 132°C. Therefore, it was found that the heating time in the heat treatment at 132°C can be obtained by putting a desired nucleotide length into Expression (II). Thus, it was found that, according to Expression (II), it is possible to determine a heating time that allows obtainment of a desired nucleotide length in a heat treatment performed at a certain heating temperature, irrespective of what heating means is used.
From the results above, it was found that, by use of the estimation method and the estimation apparatus 2 according to the present embodiment, in a case where any one or a plurality of conditions among heat treatment conditions are set to be constant, heat treatment conditions not having been set to be constant can be estimated.

**Claims**

**1.** An apparatus for estimating a heat treatment condition, the apparatus comprising:

input means for receiving an input of information of one to three heat treatment conditions selected from the group consisting of: heating time; heating temperature; kind of a buffer agent; and salt concentration of a sample which contains the nucleic acid in the sample and the buffer agent, and an input of information of a desired average nucleotide length; and
control means, wherein
on the basis of the information the inputs of which have been received by the input means, the control means estimates a heat treatment condition selected from the group consisting of: the heating time; the heating temperature; the kind of the buffer agent; and the salt concentration of the sample.

**2.** The apparatus of claim 1, wherein

the input means further receives an input of information regarding the kind of the nucleic acid.

3. The apparatus of claim 1 or 2, wherein
the input means further receives an input of information regarding a nucleotide length of nucleic acid before being subjected to the heat treatment.

4. The apparatus of any one of claims 1 to 3, wherein
the information of the heat treatment conditions includes a treatment condition library which defines correspondence relation among the kind of the buffer agent, the salt concentration of the sample, and the heating temperature or the heating time.

5. The apparatus of claim 4, wherein
on the basis of the information the inputs of which have been received by the input means, the control means outputs a constant of speed of fragmentation of nucleic acid in the heat treatment, using the treatment condition library.

6. The apparatus of claim 4 or 5, wherein
using the treatment condition library, the control means outputs information regarding a minimum nucleotide length of the nucleic acid fragments obtained in the heat treatment.

7. The apparatus of any one of claims 1 to 6, further comprising:

storage means having stored therein correspondence relation information which defines correspondence relation among the desired average nucleotide length, the heating time, the heating temperature, the kind of the buffer agent, and the salt concentration of the sample, wherein
on the basis of the information the inputs of which have been received by the input means, and on the basis of the correspondence relation information stored in the storage means, the control means estimates a heat treatment condition selected from the group consisting of: the heating time; the heating temperature; the kind of the buffer agent; and the salt concentration of the sample.

8. The apparatus of claim 7, wherein
the storage means has further stored therein a relational expression indicative of correlation among the desired average nucleotide length, the minimum nucleotide length, the nucleotide length of nucleic acid before being subjected to the heat treatment, energy for fragmentation of nucleic acid in the heat treatment, the heating temperature, and a start temperature of the heat treatment.

9. The apparatus of claim 8, wherein
the relational expression is represented by Expression (I):

$$M = A + B \times \exp\{-H(T-D)\} \qquad (I)$$

(wherein, M represents the desired average nucleotide length, A represents the minimum nucleotide length, B represents the nucleotide length of nucleic acid before being subjected to the heat treatment, H represents a constant of energy for fragmentation of nucleic acid in the heat treatment, T represents the heating temperature, and D represents the start temperature of the heat treatment).

10. The apparatus of claim 7, wherein
the storage means has further stored therein a relational expression indicative of correlation among the desired average nucleotide length, the minimum nucleotide length, the nucleotide length of nucleic acid before being subjected to the heat treatment, the speed of fragmentation of nucleic acid in the heat treatment, and the heating time.

11. The apparatus of claim 10, wherein
the relational expression is represented by Expression (II):

$$M = A + B \times \exp\{-Kt\} \qquad (II)$$

(wherein, M represents the desired average nucleotide length, A represents the minimum nucleotide length, B represents the nucleotide length of nucleic acid before being subjected to the heat treatment, K represents a constant of the speed of fragmentation of nucleic acid in the heat treatment, and t represents the heating time).

**12.** The apparatus of any one of claims 6 to 11, wherein
the storage means has further stored therein information regarding the minimum nucleotide length.

**13.** The apparatus of claim 12, wherein
the minimum nucleotide length is 10 to 80 nucleotide length.

**14.** A method for estimating a heat treatment condition, the method comprising:

on the basis of

information of one to three heat treatment conditions selected from the group consisting of: heating time; heating temperature; kind of a buffer agent; and salt concentration of a sample which contains the nucleic acid in the sample and the buffer agent, and
information of the desired average nucleotide length,

estimating a heat treatment condition selected from the group consisting of: the heating time; the heating temperature; the kind of the buffer agent; and the salt concentration of the sample, wherein the heat treatment condition is for fragmenting, through heat treatment, into the nucleic acid fragments having the desired average nucleotide length.

**15.** A computer program for estimating a heat treatment condition, the computer program for causing a computer to function as:

input means for receiving an input of information of one to three heat treatment conditions selected from the group consisting of: heating time; heating temperature; kind of a buffer agent; and salt concentration of a sample which contains the nucleic acid in the sample and the buffer agent, and an input of information of a desired average nucleotide length; and
control means for estimating a heat treatment condition selected from the group consisting of: the heating time; the heating temperature; the kind of the buffer agent; and the salt concentration of the sample, on the basis of the information the inputs of which have been received by the input means.

FIG. 1

FIG. 2

NUCLEIC ACID
FRAGMENTATION
SYSTEM

1

2

2a

```
                                    CPU ┌── 20
                              ┌─────────┐
                         21   │  CPU    │
                      ┌───────┘─────────└──────┐
              ┌──────┴──┐              ┌────────┴──┐
              │   ROM   │◄────────────►│    RAM    │  22
              └─────────┘              └───────────┘

   2b    24
┌───────┐   ┌──────────────┐              ┌──────────┐
│ INPUT │──►│ INPUT/OUTPUT │◄────────────►│          │  23
│DEVICE │   │  INTERFACE   │              │HARD DISK │
└───────┘   └──────────────┘              └──────────┘

   2c    27
            ┌──────────────┐              ┌──────────┐
         ◄──│ IMAGE OUTPUT │◄────────────►│ READOUT  │  25
            │  INTERFACE   │              │  DEVICE  │
            └──────────────┘              └──────────┘

         26
TO PRINTER ◄──┌──────────────┐
              │              │  28
              │COMMUNICATION │
              │  INTERFACE   │
┌──────────┐  │              │
│ HEATING  │◄─┤              │
│APPARATUS │  └──────────────┘
└──────────┘
```

2b

24

2c

27

26

28

TO PRINTER

HEATING
APPARATUS

3

40

STORAGE
MEDIUM

ESTIMATION APPARATUS

FIG. 3

```
              ┌──────────────┐
              │    START     │
              └──────┬───────┘
                     │
S11        ┌─────────▼──────────┐
           │ DISPLAY MODE SELECTION │
           │       SCREEN        │
           └─────────┬──────────┘
                     │                    No
S12        ╱─────────▼──────────╲──────────────────┐
           ╲   PRACTICAL MODE ?  ╱                  │
            ╲──────────┬────────╱                   │
                     │ Yes                          │
S13        ┌─────────▼──────────┐      S14 ┌────────▼────────────┐
          ││   PRACTICAL MODE   ││         ││ CONDITION PROPOSAL MODE ││
           └─────────┬──────────┘          └────────┬────────────┘
                     │◄──────────────────────────────┘
                     │                    No
S15        ╱─────────▼──────────╲──────────────┐
           ╲ EXECUTE HEAT TREATMENT ? ╱          │
            ╲──────────┬────────╱                │
                     │ Yes                       │
S16        ┌─────────▼──────────┐                │
           │   HEAT TREATMENT    │                │
           └─────────┬──────────┘                │
                     │◄──────────────────────────┘
              ┌──────▼───────┐
              │     END      │
              └──────────────┘
```

FIG. 4

```
                    ┌─────────────┐
                    │    START    │
                    └─────────────┘
                           │
                           ▼
S21  ┌────────────────────────────────────┐
     │    DISPLAY MODE SELECTION           │
     │           SCREEN                    │
     └────────────────────────────────────┘
                           │
                           ▼                          No
S22  ◁───────────────────────────────────────▷──────────────────┐
     │  TEMPERATURE-CONSTANT MODE ?        │                     │
     ◁───────────────────────────────────────▷                  │
                           │ Yes                                 │
                           ▼                                     ▼
S23  ┌┬────────────────────────────────┬┐    ┌┬──────────────────────────┬┐  S24
     ││  TEMPERATURE-CONSTANT MODE     ││    ││    TIME-CONSTANT MODE     ││
     └┴────────────────────────────────┴┘    └┴──────────────────────────┴┘
                           │                                     │
                           ▼◄────────────────────────────────────┘
                    ┌─────────────┐
                    │     END     │
                    └─────────────┘
```

FIG. 5

```
                    ┌──────────────┐
                    │    START     │
                    └──────┬───────┘
                           │
  S101                     ▼
      ┌─────────────────────────────────────┐
      │      OBTAIN EXPRESSION (II)          │
      └─────────────────────────────────────┘
                           │           ◀────────────  (B)
  S102                     ▼
      ┌─────────────────────────────────────┐
      │   REQUEST INPUT OF SET TEMPERATURE   │
      │            INFORMATION               │
      └─────────────────────────────────────┘
                           │
  S103                     ▼
      ┌─────────────────────────────────────┐
      │  OBTAIN SET TEMPERATURE INFORMATION  │
      └─────────────────────────────────────┘
                           │
  S104                     ▼
      ┌─────────────────────────────────────┐
      │    REQUEST INPUT OF INFORMATION OF   │
      │  TARGET NUCLEIC ACID, BUFFER AGENT,  │
      │             AND SAMPLE               │
      └─────────────────────────────────────┘
                           │
  S105                     ▼
      ┌─────────────────────────────────────┐
      │  OBTAIN INFORMATION OF TARGET NUCLEIC│
      │   ACID, BUFFER AGENT, AND SAMPLE     │
      └─────────────────────────────────────┘
                           │
  S106                     ▼
      ┌─────────────────────────────────────┐
      │   DETERMINE PARAMETER VALUE OF       │
      │          EXPRESSION (II)             │
      └─────────────────────────────────────┘
                           │
                           ▼
                          (A)
```

FIG. 6

500

501　　502　　503　　504　　505

| HEATING TEMPERATURE | TARGET NUCLEIC ACID | COMPONENT OF BUFFER AGENT | SAMPLE ELECTRIC CONDUCTIVITY | PARAMETER VALUE |
|---|---|---|---|---|
| 140 | DNA | ADA | ⋮ | ⋮ |
| | | PHOSPHORIC ACID | 1 | ⋮ |
| | | | 2 | |
| | | | 13 | K=2.5, A=50 |
| | | TRIS ⋮ | ⋮ | ⋮ |
| | RNA | | | |
| 160 ⋮ | ⋮ | ⋮ | ⋮ | |

FIG. 7

(A)

S107
REQUEST INPUT OF INFORMATION OF LENGTH OF TARGET NUCLEIC ACID

S108
OBTAIN INFORMATION OF LENGTH OF TARGET NUCLEIC ACID

S109
OUTPUT CORRESPONDENCE RELATION BETWEEN HEATING TIME AND NUCLEIC ACID FRAGMENT LENGTH

S110
REQUEST INPUT OF INFORMATION OF DESIRED NUCLEIC ACID FRAGMENT LENGTH

S111
OBTAIN INFORMATION OF DESIRED NUCLEIC ACID FRAGMENT LENGTH

S112
ESTIMATE HEATING TIME

S113
OUTPUT ESTIMATED HEATING TIME

S114
RE-EXECUTE ESTIMATION PROCESS ? — No

Yes

(B)

END

FIG. 8

Fragmentation shown
on the left figure is
predicted from the
inputted information.

What size of fragments
do you want ?

EP 3 091 463 A1

FIG. 9

Perform process for
1.2 min.

OK?

# FIG. 10

START

S201 — OBTAIN EXPRESSION (I)

D

S202 — REQUEST INPUT OF SET TIME INFORMATION

S203 — OBTAIN SET TIME INFORMATION

S204 — REQUEST INPUT OF INFORMATION OF TARGET NUCLEIC ACID, BUFFER AGENT, AND SAMPLE

S205 — OBTAIN INFORMATION OF TARGET NUCLEIC ACID, BUFFER AGENT, AND SAMPLE

S206 — DETERMINE PARAMETER VALUE OF EXPRESSION (I)

C

FIG. 11

(D)

S207
REQUEST INPUT OF INFORMATION OF LENGTH OF TARGET
NUCLEIC ACID

S208
OBTAIN INFORMATION OF LENGTH OF TARGET NUCLEIC
ACID

S209
OUTPUT CORRESPONDENCE RELATION BETWEEN HEATING
TEMPERATURE AND NUCLEIC ACID FRAGMENT LENGTH

S210
REQUEST INPUT OF INFORMATION OF DESIRED NUCLEIC
ACID FRAGMENT LENGTH

S211
OBTAIN INFORMATION OF DESIRED NUCLEIC ACID
FRAGMENT LENGTH

S212
ESTIMATE HEATING TEMPERATURE

S213
OUTPUT ESTIMATED HEATING TEMPERATURE

S214
RE-EXECUTE ESTIMATION PROCESS ?    No

Yes

(C)

END

FIG. 12

START

S301 — OBTAIN EXPRESSION (II)

S302 — REQUEST INPUT OF INFORMATION OF DESIRED FRAGMENTATION CURVE AND ALLOWABLE ERROR

S303 — OBTAIN INFORMATION OF DESIRED FRAGMENTATION CURVE AND ALLOWABLE ERROR

S304 — FITTING OF FRAGMENTATION CURVE TO EXPRESSION (II)

S305 — FRAGMENTATION CURVE CAN BE FITTED ? — No

Yes

S306 — OUTPUT TREATMENT CONDITION

S307 — END CONDITION PROPOSAL MODE ? — No

Yes

END

FIG. 13

801

811

NUCLEIC ACID FRAGMENT LENGTH (bp or b)

400

300

200

100

0

0          3          5

HEATING TIME (min)

What fragmentation
progress course do you
want ?

Please draw a curve
and an end point in
the left figure with
a stylus.
Please input an
allowable error, too.

812

EP 3 091 463 A1

EP 3 091 463 A1

FIG. 14

FIG. 15

700

701 702 703 704 705

| TARGET NUCLEIC ACID | PARAMETER VALUE | SAMPLE ELECTRIC CONDUCTIVITY | COMPONENT OF BUFFER AGENT | HEATING TEMPERATURE |
|---|---|---|---|---|
| DNA | ⋮ | ⋮ | ADA | 140 |
| | ⋮ | 1 | PHOSPHORIC ACID | |
| | | 2 | | |
| | K=2.5, A=70 | 13 | | |
| | ⋮ | ⋮ | TRIS ⋮ | |
| | | | | 160 ⋮ |
| RNA ⋮ | ⋮ | ⋮ | ⋮ | ⋮ |

FIG. 16

The following process
is proposed.
heating temperature:
140°C
heating time: 2.5 min
buffer agent:
phosphate buffer
electric conductivity:
13mS/cm


Perform preparatory
search again ?

EP 3 091 463 A1

FIG. 17

START

S401 — OBTAIN EXPRESSION (I)

S402 — REQUEST INPUT OF INFORMATION OF DESIRED FRAGMENTATION CURVE AND ALLOWABLE ERROR

S403 — OBTAIN INFORMATION OF DESIRED FRAGMENTATION CURVE AND ALLOWABLE ERROR

S404 — FITTING OF FRAGMENTATION CURVE TO EXPRESSION (I)

S405 — FRAGMENTATION CURVE CAN BE FITTED ?  No

Yes

S406 — OUTPUT TREATMENT CONDITION

S407 — END CONDITION PROPOSAL MODE ?  No

Yes

END

FIG. 18

FIG. 19

(A)

25°C

(B)

120°C

(C)

140°C

(D)

160°C

(E)

180°C

(F)

MARKER

STRENGTH OF BAND (arbitrary unit)

MOBILITY (arbitrary unit)

FIG. 20

FIG. 21

HEATING TEMPERATURE (°C)

FIG. 22

FIG. 23

FIG. 24

FIG. 25

EP 3 091 463 A1

FIG. 26

FIG. 27

HEATING TIME (min)

FIG. 28

Europäisches
Patentamt

European
Patent Office

Office européen
des brevets

# EUROPEAN SEARCH REPORT

**Application Number**

EP 16 16 8108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/150938 A1 (DANA FARBER CANCER INST INC [US]) 25 September 2014 (2014-09-25) <br> * abstract * <br> * page 4 - page 5 * <br> * page 17, line 8 - page 21, line 9 * <br> * claims 1, 14-16,20-22 * <br> ----- | 1-15 | INV. <br> G06F19/22 |
| A | US 2013/274146 A1 (UMBARGER MARK [US] ET AL) 17 October 2013 (2013-10-17) <br> * paragraph [0024] - paragraph [0027] * <br> ----- | 1-15 | |
| A | YU YANG ET AL: "Fragmentation of Genomic DNA using Microwave Irradiation", <br> JOURNAL OF BIOMOLECULAR TECHNIQUES, <br> 1 July 2013 (2013-07-01), XP055302631, <br> US <br> ISSN: 1524-0215, DOI: <br> 10.7171/jbt.13-2402-005 <br> * abstract * <br> * page 99, left-hand column, paragraph 4 - page 100, left-hand column * <br> ----- | 1-15 | |

**TECHNICAL FIELDS SEARCHED (IPC)**

G06F

The present search report has been drawn up for all claims

| Place of search | Date of completion of the search | Examiner |
|---|---|---|
| Munich | 15 September 2016 | Kürten, Ivayla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 16 8108

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2016

| Patent document cited in search report | | Publication date | Patent family member(s) | | Publication date |
|---|---|---|---|---|---|
| WO 2014150938 | A1 | 25-09-2014 | US | 2016032359 A1 | 04-02-2016 |
| | | | WO | 2014150938 A1 | 25-09-2014 |
| US 2013274146 | A1 | 17-10-2013 | CA | 2870702 A1 | 24-10-2013 |
| | | | EP | 2839033 A1 | 25-02-2015 |
| | | | US | 2013274146 A1 | 17-10-2013 |
| | | | WO | 2013158540 A1 | 24-10-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015096031 A

**Non-patent literature cited in the description**

- **YU YANG et al.** Fragmentation of Genomic DNA using Microwave Irradiation. *Journal of Biomolecular Techniques,* 2013, vol. 24, 98-103